# EUROPEAN PATENT APPLICATION

(11) **EP 4 303 222 A1**
(43) Date of publication of application: **10.01.2024**
(21) Application number: 22762900.3
(22) Date of filing: 04.02.2022
(51) Int. Cl.: C07F 9/09

(54) **METHOD FOR PRODUCING ASYMMETRIC PHOSPHORIC ACID TRIESTER, METHOD FOR PRODUCING SYMMETRIC PHOSPHORIC ACID TRIESTER, METHOD FOR PRODUCING PHOSPHORIC ACID ESTER, AND METHOD FOR PRODUCING ORGANOPHOSPHORUS COMPOUND**

(30) Priority: 03.03.2021 JP 2021033869
(71) Applicant: Tokyo Institute of Technology, Tokyo 152-8550 (JP); Yokogawa Electric Corporation, Musashino-shi Tokyo 180-8750 (JP)
(72) Inventor: FUSE Shinichiro, Tokyo 152-8550 (JP); KITAMURA Hiroshi, Tokyo 152-8550 (JP); OTAKE Yuma, Tokyo 152-8550 (JP); NAKAMURA Hiroyuki, Tokyo 152-8550 (JP)
(74) Representative: Hoffmann Eitle
(86) International application number: PCT/JP2022/004557
(87) International publication number: WO 2022/185843

(57) **Abstract**

A method for producing an asymmetric phosphoric acid triester using a flow reactor, the method including step (A1) of reacting phosphorus trichloride or phosphorus tribromide with a first hydroxy compound; step (B1) of reacting the compound obtained in the step (A1) with a second hydroxy compound; step (C1) of reacting the compound obtained in the step (Bl) with a third hydroxy compound; and step (Dl) of oxidizing the compound obtained in the step (C1), in which the reaction temperature in the steps (A1) and (B1) is -80°C or higher, and in the steps (A1) and (B1), the flow rate of each of the phosphorus trichloride or phosphorus tribromide, the first hydroxy compound, and the second hydroxy compound flowing in a flow channel is 0.01 mL/min or more.

## Description

### [Technical Field]

The present disclosure relates to a method for producing an asymmetric phosphoric acid triester, a method for producing a symmetric phosphoric acid triester, a method for producing a phosphoric acid ester, and a method for producing an organophosphorus compound.

Priority is claimed on Japanese Patent Application No. 2021-033869, filed on March 3, 2021, the content of which is incorporated herein by reference.

### [Background Art]

A phosphoric acid triester has a structure in which a phosphoric acid and a hydroxy compound are condensed. The phosphoric acid triester includes a symmetric phosphoric acid triester in which two identical hydroxy compounds and one different hydroxy compound are condensed, and an asymmetric phosphoric acid triester in which three different hydroxy compounds are condensed.

The symmetric phosphoric acid triester and the asymmetric phosphoric acid triester are used in various fields, such as a plasticizer, a flame retardant, and an insecticide. In addition, the asymmetric phosphoric acid triester is used as a ProTide which is a prodrug of nucleic acids, an intermediate for DNA chemical synthesis, and the like.

Examples of a method for producing an asymmetric phosphoric acid triester include a phosphoramidite method. In this method, in general, a phosphorus acid triester is obtained by a condensation reaction of a phosphoramidite with an alcohol using a tetrazole as an accelerator, and the phosphorus acid triester is converted to a phosphoric acid triester by oxidation with I₂, tert-butyl hydroperoxide, or the like.

In addition, Patent Document 1 discloses a method for producing an asymmetric phosphoric acid triesterin a non-polar solvent in the presence of a tertiary amine and at a temperature of about 10°C or lower, in which phosphorus oxychloride is added with stirring and reacted with an unsubstituted phenol or a phenol substituted with halogen or lower alkyl, the amount of the phenol being about 1.8 to 2.2 times the molar amount of phosphorus oxychloride used; and then, a monohydric alkanol which may contain a halogen or an ether bond is added to and reacted with the above reaction mixture, the amountof the monohydric alkanol being about 1 or more times the molar amount of the phosphorus oxychloride used.

### [Citation List]

### [Patent Document]

[Patent Document 1]
Japanese Unexamined Patent Application, First Publication No. S52-113949

### [Summary of Invention]

### [Technical Problem]

By the way, in the methods for producing an asymmetric phosphoric acid triester and a symmetric phosphoric acid triester, there is a demand for a method for producing a phosphoric acid triester which method is capable of producing a desired phosphoric acid triester at low cost and with high yield.

In addition, with regard to a phosphoric acid monoester and a phosphoric acid diester, which are other phosphoric acid esters, there are demands for production methods capable of producing desired phosphoric acid monoester and phosphoric acid diester at low cost and with high yield.

In addition, with regard to an organophosphorus compound, there is a demand for a production method capable of producing a desired organophosphorus compound at low cost and with high yield.

The present disclosure has been made in view of the above circumstances, and an object of the present disclosure is to provide a method for producing a phosphoric acid ester which method is capable of producing a desired phosphoric acid ester at low cost and with high yield.

In addition, the present disclosure has been made in view of the above circumstances, and an object of the present disclosure is to provide a method for producing an organophosphorus compound which method is capable of producing a desired organophosphorus compound at low cost and with high yield.

### [Solution to Problem]

In order to achieve the above-mentioned objects, the present disclosure has adopted the following configurations.

That is, a first aspect of the present disclosure is a method for producing an asymmetric phosphoric acid triester in which the asymmetric phosphoric acid triester is synthesized using a flow reactor, the method including step (A1) of reacting phosphorus trichloride or phosphorus tribromide with a first hydroxy compound represented by general formula (H-1) below to synthesize a compound P1 represented by general formula (P-1) below, step (B1) of reacting the compound P1 with a second hydroxy compound represented by general formula (H-2) below to synthesize a compound P2 represented by general formula (P-2) below, step (C1) of reacting the compound P2 with a third hydroxy compound represented by general formula (H-3) below to synthesize a compound P3 represented by general formula (P-3) below, and step (D1) of oxidizing the compound P3 to synthesize an asymmetric phosphoric acid triester, in which the reaction temperature in the steps (A1) and (B1) is -80°C or higher, and in the steps (A1) and (B1), the flow rate of each of the phosphorus trichloride or phosphorus tribromide, the first hydroxy compound, and the second hydroxy compound flowing in a flow channel is 0.01 mL/min or more. 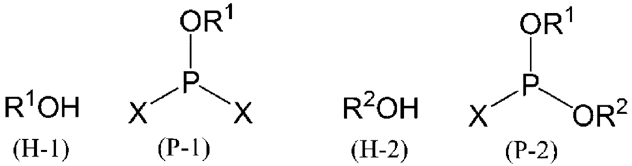 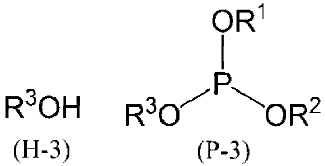 [In the formulae, R¹ to R³ are each independently an organic group. In this regard, R¹ to R³ are all different organic groups. X is a bromine atom or a chlorine atom.]

In the method for producing an asymmetric phosphoric acid triester according to the first aspect of the present disclosure, the reaction temperature in the steps (A1) and (B1) may be 0°C or higher.

In the method for producing an asymmetric phosphoric acid triester according to the first aspect of the present disclosure, in the steps (A1) and (B1), the flow rate of each of the phosphorus trichloride or phosphorus tribromide, the first hydroxy compound, and the second hydroxy compound flowing in the flow channel may be 1 mL/min or more.

The method for producing an asymmetric phosphoric acid triester according to the first aspect of the present disclosure may include step (N1) of reacting the compound P1 with a base, between the step (A1) and the step (B1).

In the method for producing an asymmetric phosphoric acid triester according to the first aspect of the present disclosure, the base may be one or more bases selected from the group consisting of pyridine, a pyridine derivative, imidazole, an imidazole derivative, and 1,4-diazabicyclo[2,2,2]octane.

In the method for producing an asymmetric phosphoric acid triester according to the first aspect of the present disclosure, the base may be a compound represented by the following general formula (I-1): 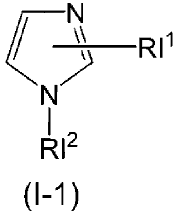 [In the formula, RI¹ and RI² are hydrogen atoms or hydrocarbon groups.]

In the method for producing an asymmetric phosphoric acid triester according to the first aspect of the present disclosure, in the step (A1), the equivalent ratio of the phosphorus trichloride or phosphorus tribromide to R¹OH (phosphorus trichloride or phosphorus tribromide:R¹OH) in a reaction system may be 0.1:1 to 10:1.
In the method for producing an asymmetric phosphoric acid triester according to the first aspect of the present disclosure, the reaction temperature in the steps (A1) and (B1) may be 20°C or higher and 40°C or lower.

In the method for producing an asymmetric phosphoric acid triester according to the first aspect of the present disclosure, in the steps (A1) and (B1), the flow rate of each of the phosphorus trichloride or phosphorus tribromide, the first hydroxy compound, and the second hydroxy compound flowing in the flow channel may be 1 mL/min or more and 5 mL/min or less.

In the method for producing an asymmetric phosphoric acid triester according to the first aspect of the present disclosure, the flow rate of phosphorus trichloride or phosphorus tribromide and the flow rate of R¹OH in the step (A1) may be different from each other.

In the method for producing an asymmetric phosphoric acid triester according to the first aspect of the present disclosure, in the step (A1), the flow rate of phosphorus trichloride or phosphorus tribromide may be 0.3 to 0.9 times the flow rate of R¹OH.

In the method for producing an asymmetric phosphoric acid triester according to the first aspect of the present disclosure, in the step (A1), the flow rate of R¹OH may be 0.3 to 0.9 times the flow rate of phosphorus trichloride or phosphorus tribromide.

A second aspect of the present disclosure is a method for producing a symmetric phosphoric acid triester in which the symmetric phosphoric acid triester is synthesized using a flow reactor, the method including step (A2) of reacting phosphorus trichloride or phosphorus tribromide with a first hydroxy compound represented by general formula (H-1) below to synthesize a compound P1 represented by general formula (P-1) below, step (B2) of reacting the compound P1 with a second hydroxy compound represented by general formula (H-2) below to synthesize a compound P4 represented by general formula (P-4) below, and step (D2) of oxidizing the compound P4 to synthesize a symmetric phosphoric acid triester, in which the reaction temperature in the steps (A2) and (B2) is -80°C or higher, and in the steps (A2) and (B2), the flow rate of each of the phosphorus trichloride or phosphorus tribromide, the first hydroxy compound, and the second hydroxy compound flowing in a flow channel is 0.01 mL/min or more. 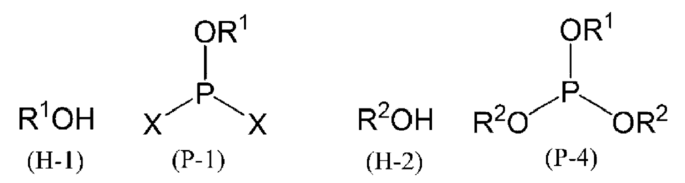 [In the formulae, R¹ and R² are organic groups which are different from each other. X is a bromine atom or a chlorine atom.]

In the method for producing a symmetric phosphoric acid triester according to the second aspect of the present disclosure, the reaction temperature in the steps (A2) and (B2) may be 0°C or higher.

In the method for producing a symmetric phosphoric acid triester according to the second aspect of the present disclosure, in the steps (A2) and (B2), the flow rate of each of the phosphorus trichloride or phosphorus tribromide, the first hydroxy compound, and the second hydroxy compound flowing in the flow channel may be 1 mL/min or more.

A third aspect of the present disclosure is a method for producing a phosphoric acid ester in which the phosphoric acid ester is synthesized using a flow reactor, the method including step (A11) of reacting phosphorus trichloride or phosphorus tribromide with a first hydroxy compound represented by general formula (H1-1) to synthesize a compound P11 represented by general formula (P1-1), step (B11) of reacting the compound P11 with a second hydroxy compound represented by general formula (H1-2) to synthesize a compound P12 represented by general formula (P1-2) below, step (C11) of reacting the compound P12 with a third hydroxy compound represented by general formula (H1-3) below to synthesize a compound P13 represented by general formula (P1-3) below, and step (D11) of oxidizing the compound P13 to synthesize a phosphoric acid ester, in which the reaction temperature in the steps (A11) and (B11) is -80°C or higher, and in the steps (A11) and (B11), the flow rate of each of the phosphorus trichloride or phosphorus tribromide, the first hydroxy compound, and the second hydroxy compound flowing in a flow channel is 0.01 mL/min or more. 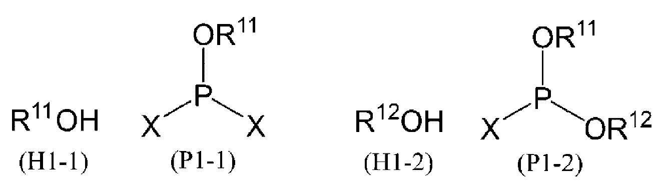 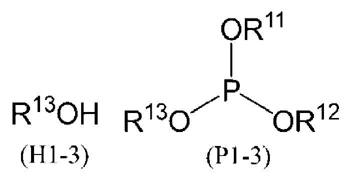 [R¹¹ to R¹³ are each independently a hydrogen atom or an organic group. In this regard, at least one of R¹¹ to R¹³ is an organic group, and at least one of R¹¹ to R¹³ is a hydrogen atom. X is a bromine atom or a chlorine atom.]

A method for producing an organophosphorus compound according to a fourth aspect of the present disclosure is a method for producing an organophosphorus compound in which the organophosphorus compound is synthesized using a flow reactor, the method including step (A01) of reacting phosphorus trichloride or phosphorus tribromide with a first hydroxy compound represented by general formula (H0-1) below to synthesize a compound P01 represented by general formula (P0-1) below, step (B01) of reacting the compound P01 with a second hydroxy compound represented by general formula (H0-2) below to synthesize a compound P02 represented by general formula (P0-2) below, and step (C01) of reacting the compound P02 with a third hydroxy compound represented by general formula (H0-3) below to synthesize a compound P03 represented by general formula (P0-3) below, in which the reaction temperature in the steps (A01) and (B01) is -80°C or higher, and in the steps (A01) and (B01), the flow rate of each of the phosphorus trichloride or phosphorus tribromide, the first hydroxy compound, and the second hydroxy compound flowing in a flow channel is 0.01 mL/min or more. 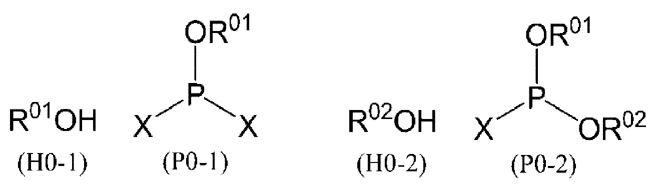 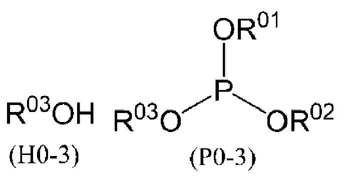 [R⁰¹ to R⁰³ are each independently a hydrogen atom or an organic group. In this regard, at least one of R⁰¹ to R⁰³ is an organic group, and in a case where all of R⁰¹ to R⁰³ are organic groups, not all three of the organic groups are the same. X is a bromine atom or a chlorine atom.]

A method for producing an organophosphorus compound according to a fifth aspect of the present disclosure is a method for producing an organophosphorus compound in which the organophosphorus compound is synthesized using a flow reactor, the method including step (AZ1) of reacting phosphorus trichloride or phosphorus tribromide with a first compound which is any of a hydroxy compound, an amine compound, or a thiol compound to synthesize a compound PZ1 represented by general formula (PZ-1) below, step (BZ1) of reacting the compound PZ1 with a second compound which is any of a hydroxy compound, an amine compound, or a thiol compound to synthesize a compound PZ2 represented by general formula (PZ-2) below, and step (CZ1) of reacting the compound PX2 with a third compound which is any of a hydroxy compound, an amine compound, or a thiol compound to synthesize a compound PZ3 represented by general formula (PZ-3) below, in which the reaction temperature in the steps (AZ1) and (BZ1) is -80°C or higher, and in the steps (AZ1) and (BZ1), the flow rate of each of the phosphorus trichloride or phosphorus tribromide, the first compound, and the second compound flowing in a flow channel is 0.01 mL/min or more. 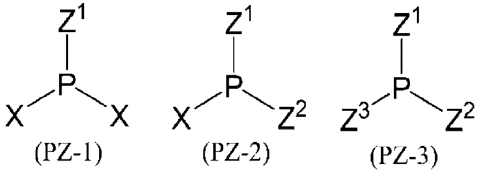 [In the formulae, Z¹ to Z³ are each independently a hydrogen atom, a group obtained by removing one hydrogen atom from a hydroxy compound, a group obtained by removing one hydrogen atom from an amine compound, or a group obtained by removing one hydrogen atom from a thiol compound. In this regard, not all three of Z¹ to Z³ are the same. X is a bromine atom or a chlorine atom.]

### [Advantageous Effects of Invention]

According to the production method of the present disclosure, a desired phosphoric acid ester can be produced at a low cost and with a high yield.

In addition, according to the production method of the present disclosure, a desired organophosphorus compound can be produced at a low cost and with a high yield.

### [Brief Description of Drawings]

FIG. 1 is a schematic diagram of a method for producing an asymmetric phosphoric acid triester according to the present embodiment.
FIG. 2 is a schematic diagram of a method for producing a symmetric phosphoric acid triester according to the present embodiment.
FIG. 3 is a schematic diagram of a method for producing a symmetric phosphoric acid triester according to Examples.
FIG. 4 is a schematic diagram of a method for producing a symmetric phosphoric acid triester according to Examples.
FIG. 5 is a schematic diagram of a method for producing an asymmetric phosphoric acid triester according to Examples.
FIG. 6 is a schematic diagram of a method for producing an asymmetric phosphoric acid triester according to Examples.

### [Description of Embodiments]

### [Brief overview]

In the methods for producing an asymmetric phosphoric acid triester and a symmetric phosphoric acid triester, there is a demand for a method for producing a phosphoric acid triester that can produce a desired phosphoric acid triester at a low cost and with a high yield.

For example, the phosphoramidite method, which is a production method in the related art, has high selectivity, but has problems such as the need to use expensive reagents, the large amount of waste, the large number of steps, and the need to use explosive tetrazole.

In addition, the method for producing an asymmetric phosphoric acid triester described in Patent Document 1 has problems such that the reaction takes a long time and the production of the desired phosphoric acid triester with high yield is difficult depending on the type of hydroxy compound used.

In order to solve the above-mentioned problems, the present inventors have made extensive studies on the method for producing a phosphoric acid ester.

As a result, it has been found that, when phosphorus trichloride or phosphorus tribromide is reacted with a first hydroxy compound in a first reaction and, then, the compound obtained in the first reaction is reacted with a second hydroxy compound in a second reaction, in case the reaction rate of the first reaction exceeds the stirring speed, there is an occurence of a side reaction in which the compound obtained in the first reaction reacts again with the first hydroxy compound used in the first reaction before carrying out the second reaction.

In view of the above, the present inventors have carried out studies using a flow reactor, and found that the desired phosphoric acid ester can be produced at a low cost and with a high yield by appropriately controlling the reaction temperature and flow rate and improving the stirring efficiency. The present disclosure has been completed based on these findings.

### <Flow reactor>

The method for producing an asymmetric phosphoric acid triester according to the first aspect of the present disclosure, the method for producing a symmetric phosphoric acid triester according to the second aspect of the present disclosure, the method for producing a phosphoric acid ester according to the third aspect of the present disclosure, and the method for producing an organophosphorus compound according to the fourth aspect of the present disclosure are production methods using a flow reactor.

The flow reactor carries out chemical reactions in a continuous flow rather than in a batch manner.

The flow reactor is usually tubular and is made of a non-reactive material. The non-reactive material is known in the related art and depends on the characteristics of raw materials or reactants. The mixing method is a diffusion method.

The residence time of the raw materials in a mixer is calculated by the mixer volume and the flow rate passing through the mixer (residence time = mixer volume/flow rate). For example, a relatively long residence time can be achieved by pumping the raw materials slowly or by using a mixer with a larger volume.

Since the mixer of the flow reactor has a very large surface area per unit volume, precise temperature control and efficient interface reaction are possible.

In addition, since the flow reactor is a flow system and makes it possible to shorten a residence time, product molecules are rapidly released out of the reactor, which can reduce the possibility of occurrence of a secondary reaction. Therefore, the flow reactor can effectively suppress the production of unwanted side reaction products.

Examples of the mixer include a T-shaped mixer and a V-shaped mixer, among which a V-shaped mixer is preferable from the viewpoint of improving the stirring efficiency.

### (Method for producing asymmetric phosphoric acid triester according to first aspect of present disclosure)

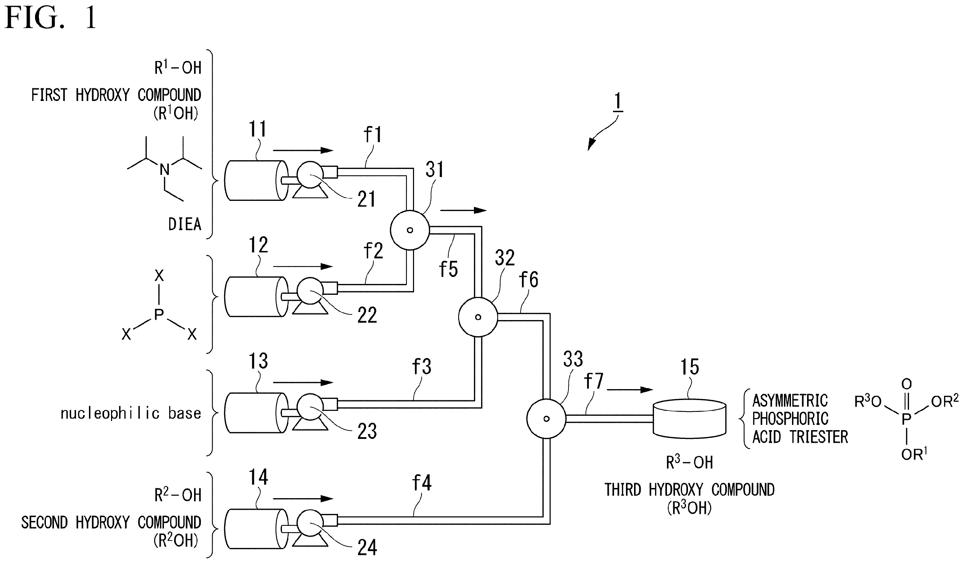

Hereinafter, the form of a flow reactor according to the present embodiment and the method for producing an asymmetric phosphoric acid triester using the flow reactor will be described with reference to FIG. 1.

FIG. 1 is a schematic diagram showing a schematic configuration of flow reactor 1. The flow reactor 1 includes tank 11 for accommodating a first liquid, tank 12 for accommodating a second liquid, tank 13 for accommodating a third liquid, tank 14 for accommodating a fourth liquid, and tank 15 for accommodating a fifth liquid.

For example, the first liquid contains a first hydroxy compound (hereinafter, also referred to as "R'OH") and N,N-diisopropylethylamine (DIEA), the second liquid contains phosphorus trichloride or phosphorus tribromide, the third liquid contains a nucleophilic base, the fourth liquid contains a second hydroxy compound (hereinafter, also referred to as "R²OH"), and the fifth liquid contains a third hydroxy compound (hereinafter, also referred to as "R³OH").

The flow reactor 1 includes flow channels f1, f2, f3, f4, f5, f6, and f7 for transporting fluids. The inner diameters of the flow channel and the mixer may be, for example, 0.1 to 1 mm, 0.1 to 0.8 mm, or 0.2 to 0.6 mm. In addition, the inner diameters of the flow channel and the mixer may be 1 mm to several cm.

The tanks 11, 12, 13, 14, and 15 and the flow channels f1, f2, f3, f4, f5, f6, and f7 are formed of, for example, resins such as plastics or elastomers, glass materials, metals, or ceramics.

The tank 11 is connected to pump 21, and the operation of the pump 21 causes the first liquid accommodated in the tank 11 to move in the flow channel f1 and flow into mixer 31. The tank 12 is connected to pump 22, and the operation of the pump 22 causes the second liquid accommodated in the tank 12 to move in the flow channel f2 and flow into the mixer 31. Next, the first liquid and the second liquid are mixed by the mixer 31 to form a first mixed liquid, which is sent to the flow channel f5. In this mixing process, R¹OH contained in the first liquid reacts with phosphorus trichloride or phosphorus tribromide contained in the second liquid to obtain compound P1 in which one chlorine atom of phosphorus trichloride or one bromine atom of phosphorus tribromide is substituted with an R¹O- group (step A1 of the method for producing an asymmetric phosphoric acid triester). The first mixed liquid containing the obtained compound P1 moves in the flow channel f5 and flows into mixer 32.

The tank 13 is connected to pump 23, and the operation of the pump 23 causes the third liquid accommodated in the tank 13 to move in the flow channel f3 and flow into the mixer 32. Next, the third liquid and the first mixed liquid are mixed to form a second mixed liquid, which is sent to the flow channel f6. In this mixing process, the compound P1 obtained in the step A1 reacts with a nucleophilic base contained in the third liquid to obtain a compound in which two chlorine atoms of the compound P1 (or two bromine atoms of the compound P1) are each substituted with a group obtained by removing one hydrogen atom from the nucleophilic base (hereinafter, also referred to as "compound N1"). The second mixed liquid containing the obtained compound N1 moves in the flow channel f6 and flows into mixer 33.

The tank 14 is connected to pump 24, and the operation of the pump 24 causes the fourth liquid accommodated in the tank 14 to move in the flow channel f4 and flow into the mixer 33. Next, the fourth liquid and the second mixed liquid are mixed to form a third mixed liquid, which is sent to the flow channel f7. In this mixing process, the compound N1 reacts with R²OH contained in the fourth liquid to obtain compound N2 in which a group obtained by removing one hydrogen atom from one nucleophilic base of the compound N1 is substituted with an R²O- group (hereinafter, also referred to as "compound N2"). The third mixed liquid containing the obtained compound N2 moves in the flow channel f7 and flows into the tank 15.

The fifth liquid containing R³OH is stored in the tank 15 and the third mixed liquid containing the compound N2 flows thereinto. Accordingly, the compound N2 reacts with R³OH to synthesize compound P3, and the compound P3 is stored in the tank 15 (step C1 of the method for producing an asymmetric phosphoric acid triester). Subsequently, an asymmetric phosphoric acid triester is synthesized by oxidizing the compound P3, and the asymmetric phosphoric acid triester is stored in the tank 15 (step D1 of the method for producing an asymmetric phosphoric acid triester).

According to the flow reactor 1 according to the present embodiment, an area for heat exchange per volume of the reaction solution can be increased. In addition, the reaction time can be controlled by the flow rate or the length of the flow channel. Therefore, the reaction solution can be strictly controlled, and as a result, the progress of unwanted side reactions can be minimized, and the yield of the desired product can be improved.

The compound P1 and the compound P2 are highly reactive, and a side reaction in which the compound P1 reacts again with R¹OH and a side reaction in which the compound P2 reacts again with R²OH are likely to occur, so that it is important to control the reaction.

According to the flow reactor 1 according to the present embodiment, the synthesized compound P1 and compound P2 can be rapidly released from the mixer, and thus the side reactions can be suppressed, making it possible to produce the desired phosphoric acid triester with high yield.

The flow reactor 1 has been exemplified as a form in which the liquids are mixed by a mixer, but the mixing of the liquids can be achieved only by communication between the flow channels, so the flow reactor of the present embodiment does not necessarily have a mixer.

### <First embodiment>

The method for producing an asymmetric phosphoric acid triester of the first embodiment is a method for producing an asymmetric phosphoric acid triester in which the asymmetric phosphoric acid triester is synthesized using a flow reactor, the method including step (A1) of reacting phosphorus trichloride or phosphorus tribromide with a first hydroxy compound to synthesize compound P1 (hereinafter, referred to as "step (A1)"), step (B1) of reacting the compound P1 with a second hydroxy compound to synthesize compound P2 (hereinafter, referred to as "step (B1)"), step (C1) of reacting the compound P2 with a third hydroxy compound to synthesize compound P3 (hereinafter, referred to as "step (C1)"), and step (D1) of oxidizing the compound P3 to synthesize an asymmetric phosphoric acid triester (hereinafter, referred to as "step (D1)").

### <<Step (A1)>>

The step (A1) is a step of reacting phosphorus trichloride or phosphorus tribromide with a first hydroxy compound (R¹OH) represented by general formula (H-1) below to synthesize compound P1 represented by general formula (P-1) below. 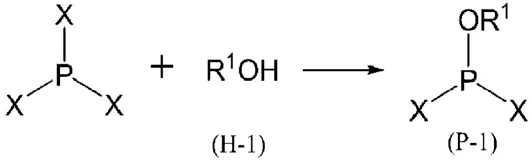 [In the formulae, R¹ is an organic group. X is a bromine atom or a chlorine atom.]

In the general formulae (H-1) and (P-1), R¹ is an organic group.

Examples of the organic group include a hydrocarbon group which may have a substituent.

The hydrocarbon group may be linear, branched, or cyclic. In a case where the hydrocarbon group is a cyclic hydrocarbon group, the cyclic hydrocarbon group may be an aliphatic hydrocarbon group or an aromatic hydrocarbon group, and may be a polycyclic group or a monocyclic group.

The substituent is not particularly limited, and examples thereof include an alkoxy group, a halogen atom, a halogenated alkyl group, a carboxy group, a cyano group, and an amino group.

In addition, some of the carbon atoms in the hydrocarbon group may be substituted with a substituent containing a hetero atom. Examples of the substituent containing a hetero atom include -O-, -C(=O)-O-, -S-, -S(=O)₂-, and -S(=O)₂-O-.

More specifically, R¹ is preferably a group obtained by removing one hydroxy group from the first hydroxy compound which will be described below.

### [First hydroxy compound (R'OH)]

The first hydroxy compound (R¹OH) is not particularly limited as long as it is a compound having one or more hydroxy groups, and a known compound can be used. R¹OH may have a substituent other than the hydroxy group.

Specific examples of R¹OH include monool, polyalkylene glycol monoether, polyalkylene glycol monoester, polyol, sugar, glycerin fatty acid ester, ethanolamide, and nucleoside.

### · Monool

More specific examples of the monool include a monohydric aliphatic alcohol and a monohydric aromatic alcohol.

### ·· Aliphatic alcohol

The monohydric aliphatic alcohol is a compound having one hydroxy group in a linear, branched, or cyclic aliphatic hydrocarbon group.

For example, the monohydric aliphatic alcohol is preferably an aliphatic alcohol having 1 to 20 carbon atoms, more preferably an aliphatic alcohol having 1 to 10 carbon atoms, and still more preferably an aliphatic alcohol having 1 to 7 carbon atoms.

Specific examples of the monohydric aliphatic alcohol include saturated aliphatic alcohols, such as methanol, ethanol, 1-propanol, 2-propanol, 1-butanol (n-butanol), 2-methylpropyl alcohol (isobutanol), 2-butanol (sec-butanol), 2-methyl-2-propanol (tert-butanol), 1-pentanol (n-amyl alcohol), 2-pentanol (sec-amyl alcohol), 3-pentanol, 2-methyl-1-butanol, 3-methyl-1-butanol (isoamyl alcohol), 2-methyl-2-butanol (tert-amyl alcohol), 3-methyl-2-butanol, 2,2-dimethyl-1-propanol (neopentyl alcohol), tert-butyl hydroxyacetate (tert-butyl glycolate), 2-[[tert-butyl(dimethyl)silyl]oxy]ethanol, 2-methoxy-ethan-1-ol (ethylene glycol monomethyl ether), tert-butyl(2-hydroxyethyl)carbamate, and 2-cyanoethanol; and unsaturated aliphatic alcohols, such as 2-propen-1-ol (allyl alcohol), 3-buten-1-ol, 4-penten-1-ol, 5-hexen-1-ol, 6-hepten-1-ol, and propargyl alcohol.

### ·· Monohydric aromatic alcohol

The monohydric aromatic alcohol is a compound having one hydroxy group and one or more aromatic rings. The aromatic ring is not particularly limited as long as it is a cyclic conjugate having (4n + 2) π electrons, and may be monocyclic or polycyclic.

For example, the monohydric aromatic alcohol is preferably an aromatic alcohol having 6 to 30 carbon atoms and more preferably an aromatic alcohol having 6 to 15 carbon atoms.

Specific examples of the monohydric aromatic alcohol include phenol, cresol, ethylphenol, tert-butylphenol, hexylphenol, octylphenol, nonylphenol, decylphenol, undecylphenol, dodecylphenol, tridecylphenol, tetradecylphenol, phenylphenol, benzylphenol, styrenated phenol, p-cumylphenol, 2-phenylethanol (phenethyl alcohol), and 2-hydroxyindane (2-indanol).

### · Polyalkylene glycol monoether

Examples of the polyalkylene glycol monoether include compounds obtained by adding alkylene oxides, such as ethylene oxide, propylene oxide, butylene oxide, and long-chain α-olefin oxides to the above-mentioned monools.

Specific examples of the polyalkylene glycol monoether include polyoxyethylene monooctyl ether, polyoxyethylene/polyoxypropylene monooctyl ether, polyoxyethylene monodecyl ether, polyoxyethylene/polyoxypropylene monodecyl ether, polyoxyethylene monolauryl ether, polyoxyethylene/polyoxypropylene monolauryl ether, polyoxyethylene monomyristyl ether, polyoxyethylene/polyoxypropylene monomyristyl ether, polyoxyethylene monopalmityl ether, polyoxyethylene/polyoxypropylene monopalmityl ether, polyoxyethylene monostearyl ether, polyoxyethylene/polyoxypropylene monostearyl ether, polyoxyethylene monooleyl ether, polyoxyethylene/polyoxypropylene monooleyl ether, polyoxyethylene mono coconut alkyl ether, polyoxyethylene/polyoxypropylene mono coconut alkyl ether, polyoxyethylene mono palm alkyl ether, polyoxyethylene/polyoxypropylene mono palm alkyl ether, polyoxyethylene mono beef tallow alkyl ether, and polyoxyethylene/polyoxypropylene mono beef tallow alkyl ether.

### · Polyalkylene glycol monoester

Examples of the polyalkylene glycol monoester include compounds obtained by adding alkylene oxides to fatty acids, and compounds obtained by subjecting polyalkylene glycols and fatty acids to an esterification reaction.

Specific examples of the polyalkylene glycol monoester include polyethylene glycol monolaurate, polyethylene/polypropylene glycol monolaurate, polyethylene glycol myristate, polyethylene/polypropylene glycol monomyristate, polyethylene glycol palmitate, polyethylene/polypropylene glycol monopalmitate, polyethylene glycol monostearate, polyethylene/polypropylene glycol monostearate, polyethylene glycol monooleate, polyethylene/polypropylene glycol monooleate, polyethylene glycol mono coconut fatty acid ester, polyethylene/polypropylene glycol mono coconut fatty acid ester, polyethylene glycol mono palm fatty acid ester, polyethylene/polypropylene glycol mono palm fatty acid ester laurate, polyethylene glycol mono beef tallow fatty acid ester, and polyethylene/polypropylene glycol mono beef tallow fatty acid ester.

### · Polyol

The polyol is a compound having two or more hydroxy groups.

Specific examples of the polyol include dihydric alcohols, such as ethylene glycol, propylene glycol, 1,3-propanediol, 1,4-butanediol, 1,5-pentanediol, 1,6-hexanediol, 1,7-heptanediol, 1,8-octanediol, 1,9-nonanediol, 1,10-decanediol, 1,2-propanediol, 2-methyl-1,3-propanediol, 1,3-butanediol, 2-methyl-1,4-butanediol, 1,4-pentanediol, 2-methyl-1,5-pentanediol, 3-methyl-1,5-pentanediol, 1,5-hexanediol, 2-methyl-1,6-hexanediol, 3-methyl-1,6-hexanediol, 1,6-heptanediol, 2-methyl-1,7-heptanediol, 3-methyl-1,7-heptanediol, 4-methyl-1,7-heptanediol, 1,7-octanediol, 2-methyl-1,8-octanediol, 3-methyl-1,8-octanediol, 4-methyl-1,8-octanediol, 1,8-nonanediol, 2-methyl-1,9-nonanediol, 3-methyl-1,9-nonanediol, 4-methyl-1,9-nonanediol, 5-methyl-1,9-nonanediol, 2-ethyl-1,3-hexanediol, 2,4-diethyl-1,5-pentanediol, neopentyl glycol, 2,2-diethylpropanediol, and 2-butyl-2-ethylpropananediol; trihydric alcohols such as glycerin, trioxyisobutane, 1,2,3-butanetriol, 1,2,3-pentanetriol, 2-methyl-1,2,3-propanetriol, 2-methyl-2,3,4-butanetriol, 2-ethyl-1,2,3-butanetriol, 2,3,4-pentanetriol, 2,3,4-hexanetriol, 4-propyl-3,4,5-heptanetriol, 2,4-dimethyl-2,3,4-pentanetriol, pentamethylglycerin, pentaglycerin, 1,2,4-butanetriol, 1,2,4-pentanetriol, trimethylolethane, and trimethylolpropane; tetrahydric alcohols, such as pentaerythritol, erythritol, 1,2,3,4-pentanetetrol, 2,3,4,5-hexanetetrol, 1,2,4,5-pentanetetrol, 1,3,4,5-hexanetetrol, diglycerin, and sorbitan; pentahydric alcohols, such as adonitol, arabitol, xylitol, and triglycerin; hexahydric alcohols such as dipentaerythritol, sorbitol, mannitol, iditol, inositol, dulcitol, talose, and allose; and derivatives of the polyols in which some or all of the hydrogen atoms of those polyol compounds are substituted with other substituents.

Taking inositol as an example, there are nine stereoisomers of inositol: cis-inositol, epi-inositol, allo-inositol, myo-inositol, muco-inositol, neo-inositol, chiro-inositol (D-form and L-form), and scyllo-inositol. Similarly, for other compounds, only one isomer may be used, or two or more isomers may be used in combination.

### · Sugar

The sugar may be a monosaccharide or an oligosaccharide. Here, the monosaccharide refers to a sugar that cannot be further hydrolyzed, and refers to a compound that is a constituent element when forming a polysaccharide. The monosaccharide can also be said to be the smallest unit of a sugar. The oligosaccharide is an oligomer of sugars in which a plurality of monosaccharides are bonded by glycoside bonds.

### ·· Monosaccharide

Specific examples of the monosaccharide include glucose, fructose, galactose, ribose, xylose, mannitol, sorbitol, xylitol, erythritol, and pentaerythritol.

### ·· Oligosaccharide

Specific examples of the oligosaccharide include disaccharides, such as sucrose, lactose, maltose, isomaltose, trehalose, cellobiose, and maltitol; trisaccharides, such as raffinose, melezitose, and maltotriose; tetrasaccharides, such as stachyose; hexasaccharides such as α-cyclodextrin; heptasaccharides, such as β-cyclodextrin; and octasaccharides, such as γ-cyclodextrin.

### ·· Other sugars

In addition, examples of the sugar may include other sugars, such as heptoses, deoxy sugars, amino sugars, thio sugars, seleno sugars, aldonic acid, uronic acid, sugar acids, ascorbic acid, ketoaldonic acid, anhydro sugars, unsaturated sugars, sugar esters, sugar ethers, and glycosides, and may include hydrolyzates of polysaccharides, such as starch, glycogen, and cellulose.

### · Glycerin fatty acid ester

The glycerin fatty acid ester is a compound in which a fatty acid is ester-bonded to one or two of the three hydroxy groups of glycerin.

Specific examples of the glycerin fatty acid ester include glycerin monolaurate, glycerin monomyristate, glycerin monopalmitate, glycerin monostearate, glycerin monooleate, glycerin mono coconut fatty acid ester, glycerin mono palm fatty acid ester, and glycerin mono beef tallow fatty acid ester.

### · Ethanolamide

Examples of the ethanolamide include fatty acid amides of monoethanolamine or diethanolamine, and compounds obtained by further adding alkylene oxides to the fatty acid amides.

Specific examples of the ethanolamide include lauric acid monoethanolamide, lauric acid diethanolamide, a lauric acid monoethanolamide ethylene oxide adduct, a lauric acid diethanolamide ethylene oxide adduct, myristic acid monoethanolamide, myristic acid diethanolamide, a myristic acid monoethanolamide ethylene oxide adduct, a myristic acid diethanolamide ethylene oxide adduct, palmitic acid monoethanolamide, palmitic acid diethanolamide, a palmitic acid monoethanolamide ethylene oxide adduct, a palmitic acid diethanolamide ethylene oxide adduct, stearic acid monoethanolamide, stearic acid diethanolamide, a stearic acid monoethanolamide ethylene oxide adduct, a stearic acid diethanolamide ethylene oxide adduct, oleic acid monoethanolamide, oleic acid diethanolamide, an oleic acid monoethanolamide ethylene oxide adduct, an oleic acid diethanolamide ethylene oxide adduct, coconut fatty acid monoethanolamide, coconut fatty acid diethanolamide, a coconut fatty acid monoethanolamide ethylene oxide adduct, a coconut fatty acid diethanolamide ethylene oxide adduct, palm fatty acid monoethanolamide, palm fatty acid diethanolamide, a palm fatty acid monoethanolamide ethylene oxide adduct, a palm fatty acid diethanolamide ethylene oxide adduct, beef tallow fatty acid monoethanolamide, beef tallow fatty acid diethanolamide, a beef tallow fatty acid monoethanolamide ethylene oxide adduct, and a beef tallow fatty acid diethanolamide ethylene oxide adduct.

### · Nucleoside

The nucleoside is a compound in which a base and a sugar are bonded. Examples of the base include purine bases, such as adenine and guanine; pyrimidine bases, such as thymine, cytosine, and uracil; nicotinamide; and dimethyl isoalloxazine.

Specific examples of the nucleoside include adenosine, guanosine, 5-methyluridine, uridine, cytidine, deoxyadenosine, deoxyguanosine, deoxyguanosine, thymidine, deoxyuridine, and deoxycitidine. Further examples of the nucleoside include nucleoside derivatives in which some or all of the hydrogen atoms or hydroxy groups of those compounds are substituted with other substituents (for example, 5-(3-benzoyl-5-methyl-2,4-dioxo-3,4-dihydropyrimidin-1(2H)-yl)-2-(hydroxymethyl)tetrahydrofuran-3-ylbenzoate, and 3-benzoyl-1-[4-hydroxy-5-(tert-butyldimethylsilyloxymethyl)tetrahydrofuran-2-yl)-5-methylpyrimidine-2,4(1H,3H)-dione).

R¹OH may be a compound other than the above-mentioned monool, polyalkylene glycol monoether, polyalkylene glycol monoester, polyol, sugar, glycerin fatty acid ester, ethanolamide, and nucleoside, and may be, for example, a hydroxyamino acid, such as threonine or serine, or a derivative thereof (for example, methyl[[(9H-fluorenyl)methoxy]carbonyl]serinate); or a hydroxy acid, such as glycolic acid, lactic acid, malic acid, tartaric acid, citric acid, or ricinoleic acid.

Among the above, R ¹OH is preferably a monool, an amino acid (including an amino acid derivative), or a nucleoside (including a nucleoside derivative).

The monool is preferably an aliphatic alcohol having 1 to 20 carbon atoms or an aromatic alcohol having 6 to 30 carbon atoms, and more preferably an aliphatic alcohol having 1 to 7 carbon atoms or an aromatic alcohol having 6 to 15 carbon atoms.

More specifically, the monool as R¹OH is preferably ethanol, 1-butanol, 2-methylpropyl alcohol, 2-butanol, 2-methyl-2-propanol, 1-pentanol, 2-pentanol, 3-pentanol, 2-methyl-1-butanol, 3-methyl-1-butanol, 2-methyl-2-butanol, 3-methyl-2-butanol, 2,2-dimethyl-1-propanol, tert-butyl hydroxyacetate, 2-[[tert-butyl(dimethyl)silyl]oxy]ethanol, 2-propen-1-ol, 3-buten-1-ol, 4-penten-1-ol, 5-hexen-1-ol, 6-hepten-1-ol, phenol, cresol, ethylphenol, tert-butylphenol, hexylphenol, octylphenol, nonylphenol, decylphenol, undecylphenol, dodecylphenol, tridecylphenol, tetradecylphenol, phenylphenol, benzylphenol, styrenated phenol, p-cumylphenol, 2-phenylethanol, or 2-hydroxyindane, and more preferably ethanol, 1-butanol (n-butanol), 2-methylpropyl alcohol (isobutanol), 2-butanol (sec-butanol), 2-methyl-2-propanol (tert-butanol), tert-butyl hydroxyacetate (tert-butyl glycolate), 2-[[tert-butyl(dimethyl)silyl]oxy]ethanol, 2-propen-1-ol (allyl alcohol), 3-buten-1-ol, 4-penten-1-ol, phenol, 2-phenylethanol (phenethyl alcohol), 2-hydroxyindane (2-indanol), 2-methoxy-ethan-1-ol, propargyl alcohol, tert-butyl(2-hydroxyethyl)carbamate, or 2-cyanoethanol.

The amino acid (including an amino acid derivative) as R¹OH is preferably methyl[[(9H-fluorenyl)methoxy]carbonyl]serinate.

The nucleoside (including a nucleoside derivative) as R¹OH is preferably 5-(3-benzoyl-5-methyl-2,4-dioxo-3,4-dihydropyrimidin-1(2H)-yl)-2-(hydroxymethyl)tetrahydrofuran-3-ylbenzoate, or 3-benzoyl-1-[4-hydroxy-5-(tert-butyldimethylsilyloxymethyl)tetrahydrofuran-2-yl)-5-methylpyrimidine-2,4(1H,3H)-dione.

In the step (A1), the amount of each of phosphorus trichloride or phosphorus tribromide and R¹OH to be used may be appropriately adjusted according to the intended reaction in consideration of the type of R¹OH to be used.

The equivalent ratio of phosphorus trichloride or phosphorus tribromide to R¹OH (phosphorus trichloride or phosphorus tribromide:R¹OH) in the reaction system may be 0.1:1 to 10:1, 0.2:1 to 5:1, or 0.5:1 to 3:1.

According to the step (A1) in the method for producing an asymmetric phosphoric acid triester of the present embodiment, the compound P1 can be synthesized in a high yield even in a case where R¹OH is reacted with a relatively small amount of phosphorus trichloride or phosphorus tribromide, which is close to an equal equivalent.

The lower limit value of the reaction temperature in the step (A1) is -80°C or higher, preferably -10°C or higher, more preferably 0°C or higher, still more preferably 10°C or higher, and particularly preferably 20°C or higher.

In addition, the upper limit value of the reaction temperature in the step (A1) is not particularly limited, and is, for example, preferably 80°C or lower, more preferably 60°C or lower, and still more preferably 40°C or lower.

In a case where the lower limit value of the reaction temperature in the step (A1) is -80°C or higher, the stirring efficiency can be improved and side reactions can be further suppressed. In addition, in a case where the lower limit value of the reaction temperature in the step (A1) is equal to or more than the above-mentioned preferred lower limit value, the stirring efficiency can be further improved and side reactions can be further suppressed, which makes it possible to further improve the yield of the compound P1.

In a case where the upper limit value of the reaction temperature in the step (A1) is equal to or less than the above-mentioned preferred upper limit value, the reaction rate of phosphorus trichloride or phosphorus tribromide with R¹OH can be appropriately controlled and side reactions can be further suppressed.

For example, the reaction temperature in the step (A1) is preferably -80°C or higher and 80°C or lower, more preferably -10°C or higher and 80°C or lower, still more preferably 0°C or higher and 60°C or lower, particularly preferably 10°C or higher and 40°C or lower, and most preferably 20°C or higher and 40°C or lower.

In the step (A1), the lower limit value of the flow rate of phosphorus trichloride or phosphorus tribromide flowing in the flow channel is 0.01 mL/min or more, preferably 0.1 mL/min or more, and more preferably 1 mL/min or more.

In addition, in the step (A1), the upper limit value of the flow rate of phosphorus trichloride or phosphorus tribromide flowing in the flow channel is not particularly limited, and may be, for example, 10 mL/min or less, 8 mL/min or less, or 5 mL/min or less.

In the step (A1), in a case where the lower limit value of the flow rate of phosphorus trichloride or phosphorus tribromide flowing in the flow channel is 0.01 mL/min or more, the stirring efficiency can be improved and side reactions can be further suppressed. In addition, in the step (A1), in a case where the lower limit value of the flow rate of phosphorus trichloride or phosphorus tribromide flowing in the flow channel is equal to or more than the above-mentioned preferred lower limit value, the stirring efficiency can be further improved and side reactions can be further suppressed, which makes it possible to further improve the yield of the compound P1.

In addition, in the step (A1), in a case where the upper limit value of the flow rate of phosphorus trichloride or phosphorus tribromide flowing in the flow channel is equal to or less than the above-mentioned preferred upper limit value, the reaction rate of phosphorus trichloride or phosphorus tribromide with R¹OH can be appropriately controlled and side reactions can be further suppressed.

For example, in the step (A1), the flow rate of phosphorus trichloride or phosphorus tribromide flowing in the flow channel is preferably 0.01 mL/min or more and 10 mL/min or less, more preferably 0.1 mL/min or more and 8 mL/min or less, and still more preferably 1 mL/min or more and 5 mL/min or less.

In the step (A1), the lower limit value of the flow rate of R¹OH flowing in the flow channel is 0.01 mL/min or more, preferably 0.1 mL/min or more, more preferably 1 mL/min or more, and still more preferably 2 mL/min or more.

In addition, in the step (A1), the upper limit value of the flow rate of R¹OH flowing in the flow channel is not particularly limited, and may be, for example, 10 mL/min or less, 8 mL/min or less, or 5 mL/min or less.

In the step (A1), in a case where the lower limit value of the flow rate of R¹OH flowing in the flow channel is 0.01 mL/min or more, the stirring efficiency can be improved and side reactions can be further suppressed. In addition, in the step (A1), in a case where the lower limit value of the flow rate of R¹OH flowing in the flow channel is equal to or more than the above-mentioned preferred lower limit value, the stirring efficiency can be further improved and side reactions can be further suppressed, which makes it possible to further improve the yield of the compound P1.

In addition, in the step (A1), in a case where the upper limit value of the flow rate of R¹OH flowing in the flow channel is equal to or less than the above-mentioned preferred upper limit value, the reaction rate of phosphorus trichloride or phosphorus tribromide with R¹OH can be appropriately controlled and side reactions can be further suppressed.

For example, in the step (A1), the flow rate of R¹OH flowing in the flow channel is preferably 0.01 mL/min or more and 10 mL/min or less, more preferably 0.1 mL/min or more and 8 mL/min or less, still more preferably 1 mL/min or more and 5 mL/min or less, and particularly preferably 2 mL/min or more and 5 mL/min or less.

In the step (A1), the flow rate of phosphorus trichloride or phosphorus tribromide and the flow rate of R¹OH may be different or the same, and are preferably different.

Specifically, either one of the above flow rates is preferably 0.1 to 1.0 times and more preferably 0.3 to 0.9 times the other flow rate.

For example, the flow rate of phosphorus trichloride or phosphorus tribromide may be 1 mL/min or more and 5 mL/min or less, and the flow rate of R¹OH may be 0.3 to 0.9 times the flow rate of the phosphorus trichloride or phosphorus tribromide, or the flow rate of phosphorus trichloride or phosphorus tribromide may be 2 mL/min or more and 5 mL/min or less, and the flow rate of R¹OH may be 0.3 to 0.9 times the flow rate of the phosphorus trichloride or phosphorus tribromide.

In addition, on the contrary, the flow rate of R¹OH may be 1 mL/min or more and 5 mL/min or less, and the flow rate of phosphorus trichloride or phosphorus tribromide may be 0.3 to 0.9 times the flow rate of the R'OH, or the flow rate of R¹OH may be 2 mL/min or more and 5 mL/min or less, and the flow rate of phosphorus trichloride or phosphorus tribromide may be 0.3 to 0.9 times the flow rate of the R¹OH.

In the step (A1), the reaction time for reacting phosphorus trichloride or phosphorus tribromide with R¹OH in the mixer of the flow reactor is not particularly limited, and is, for example, 0.01 seconds to 1 minute, and may be 1 to 30 seconds or 1 to 20 seconds.

The compound P1 synthesized in the step (A1) is a compound in which one chlorine atom of phosphorus trichloride or one bromine atom of phosphorus tribromide is substituted with a group obtained by removing one hydrogen atom from the hydroxy group of R¹OH.

For example, the compound P1 is a compound in which one chlorine atom of phosphorus trichloride or one bromine atom of phosphorus tribromide is substituted with a group obtained by removing one hydrogen atom from the hydroxy group of the above-mentioned monool, polyalkylene glycol monoether, polyalkylene glycol monoester, polyol, sugar, glycerin fatty acid ester, ethanolamide, or nucleoside.

### <<Step (B1)>>

The step (B1) is a step of reacting the above-mentioned compound P1 with a second hydroxy compound (R²OH) to synthesize compound P2 represented by the following general formula (P-2): 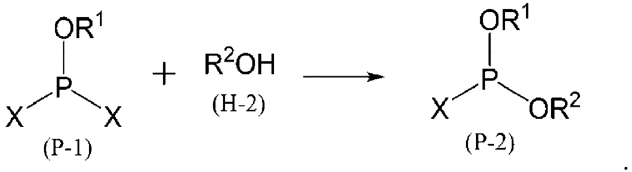 [In the formulae, R¹ and R² are organic groups which are different from each other. X is a bromine atom or a chlorine atom.]

In the general formulae (H-2) and (P-2), R² is an organic group, and examples thereof include the same groups as those for R¹ described above. In this regard, R¹ and R² are organic groups which are different from each other.

Examples of R²OH include the same compounds as those for R¹OH described above, but R²OH is not the same compound as R¹OH.

The lower limit value of the reaction temperature in the step (B1) is -80°C or higher, preferably -10°C or higher, more preferably 0°C or higher, still more preferably 10°C or higher, and particularly preferably 20°C or higher.

In addition, the upper limit value of the reaction temperature in the step (B1) is not particularly limited, and is, for example, preferably 80°C or lower, more preferably 60°C or lower, and still more preferably 40°C or lower.

In a case where the lower limit value of the reaction temperature in the step (B1) is -80°C or higher, the stirring efficiency can be improved and side reactions can be further suppressed. In addition, in a case where the lower limit value of the reaction temperature in the step (B1) is equal to or more than the above-mentioned preferred lower limit value, the stirring efficiency can be further improved and side reactions can be further suppressed, which makes it possible to further improve the yield of the compound P2.

In a case where the upper limit value of the reaction temperature in the step (B1) is equal to or less than the above-mentioned preferred upper limit value, the reaction rate of the compound P1 with R²OH can be appropriately controlled and side reactions can be further suppressed.

For example, the reaction temperature in the step (B1) is preferably -80°C or higher and 80°C or lower, more preferably -10°C or higher and 80°C or lower, still more preferably 0°C or higher and 60°C or lower, particularly preferably 10°C or higher and 40°C or lower, and most preferably 20°C or higher and 40°C or lower.

In the step (B1), the lower limit value of the flow rate of R²OH flowing in the flow channel is 0.01 mL/min or more, preferably 0.1 mL/min or more, more preferably 1 mL/min or more, and still more preferably 2 mL/min or more.

In addition, in the step (B1), the upper limit value of the flow rate of R²OH flowing in the flow channel is not particularly limited, and may be, for example, 10 mL/min or less, 8 mL/min or less, or 5 mL/min or less.

In the step (B1), in a case where the lower limit value of the flow rate of R²OH flowing in the flow channel is 0.01 mL/min or more, the stirring efficiency can be improved and side reactions can be further suppressed. In addition, in the step (B1), in a case where the lower limit value of the flow rate of R²OH flowing in the flow channel is equal to or more than the above-mentioned preferred lower limit value, the stirring efficiency can be further improved and side reactions can be further suppressed, which makes it possible to further improve the yield of the compound P2.

In addition, in the step (B1), in a case where the upper limit value of the flow rate of R²OH flowing in the flow channel is equal to or less than the above-mentioned preferred upper limit value, the reaction rate of the compound P1 with R²OH can be appropriately controlled and side reactions can be further suppressed.

For example, in the step (B1), the flow rate of R²OH flowing in the flow channel is preferably 0.01 mL/min or more and 10 mL/min or less, more preferably 0.1 mL/min or more and 8 mL/min or less, still more preferably 1 mL/min or more and 5 mL/min or less, and particularly preferably 2 mL/min or more and 5 mL/min or less.

In the step (B1), the reaction time for reacting the compound P1 with R²OH in the mixer of the flow reactor is not particularly limited, and is, for example, 0.01 seconds to 1 minute, and may be 1 to 30 seconds or 1 to 20 seconds.

The compound P2 synthesized in the step (B1) is a compound in which one chlorine atom of the compound P1 (or one bromine atom of the compound P1) is substituted with a group obtained by removing one hydrogen atom from the hydroxy group of R²OH. In addition, the compound P2 can also be said to be a compound in which one chlorine atom of phosphorus trichloride (or one bromine atom of phosphorus tribromide) is substituted with a group obtained by removing one hydrogen atom from the hydroxy group of R'OH, and another chlorine atom of phosphorus trichloride (or another bromine atom of phosphorus tribromide) is substituted with a group obtained by removing one hydrogen atom from the hydroxy group of R²OH.

For example, the compound P2 is a compound in which one chlorine atom of phosphorus trichloride or one bromine atom of phosphorus tribromide is substituted with a group (HG1) obtained by removing one hydrogen atom from the hydroxy group of the above-mentioned monool, polyalkylene glycol monoether, polyalkylene glycol monoester, polyol, sugar, glycerin fatty acid ester, ethanolamide, or nucleoside, and another chlorine atom of phosphorus trichloride or another bromine atom of phosphorus tribromide is substituted with a group (HG2) obtained by removing one hydrogen atom from the hydroxy group of the above-mentioned monool, polyalkylene glycol monoether, polyalkylene glycol monoester, polyol, sugar, glycerin fatty acid ester, ethanolamide, or nucleoside (provided that HG1 and HG2 are different substituent from each other).

### «Step (C1)»

The step (C1) is a step of reacting the above-mentioned compound P2 with a third hydroxy compound (R³OH) to synthesize a compound P3 represented by the following general formula (P-3). 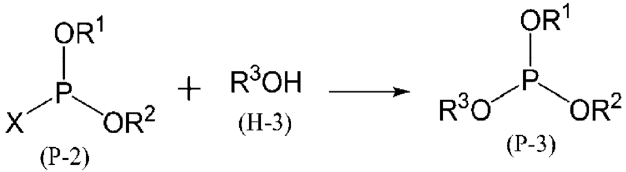 [In the formulae, R¹ to R³ are each independently an organic group. In this regard, all of R¹ to R³ are different organic groups. X is a bromine atom or a chlorine atom.]

In the general formulae (H-3) and (P-3), R³ is an organic group, examples of which include the same groups as those for R¹ described above. In this regard, R¹ to R³ are all different organic groups.

Examples of R³OH include the same compounds as those for R¹OH described above, but R³OH is not the same compound as R²OH and R¹OH.

In the step (C1), as described above, a compound P3 can be synthesized by, for example, storing R³OH in a tank and allowing the above-mentioned compound P2 to flow into the tank.

The reaction temperature in the step (C1) is not particularly limited, and is preferably -80°C or higher and 80°C or lower, more preferably -10°C or higher and 80°C or lower, still more preferably 0°C or higher and 60°C or lower, particularly preferably 10°C or higher and 40°C or lower, and most preferably 20°C or higher and 40°C or lower.

The compound P3 synthesized in the step (C1) is a compound in which one chlorine atom of the compound P2 (or one bromine atom of the compound P2) is substituted with a group obtained by removing one hydrogen atom from the hydroxy group of R³OH.

For example, the compound P3 is a compound in which one chlorine atom of phosphorus trichloride or one bromine atom of phosphorus tribromide is substituted with a group (HG1) obtained by removing one hydrogen atom from the hydroxy group of the above-mentioned monool, polyalkylene glycol monoether, polyalkylene glycol monoester, polyol, sugar, glycerin fatty acid ester, ethanolamide, or nucleoside, another chlorine atom of phosphorus trichloride or another bromine atom of phosphorus tribromide is substituted with a group (HG2) obtained by removing one hydrogen atom from the hydroxy group of the above-mentioned monool, polyalkylene glycol monoether, polyalkylene glycol monoester, polyol, sugar, glycerin fatty acid ester, ethanolamide, or nucleoside, and the remaining one chlorine atom of phosphorus trichloride or the remaining one bromine atom of phosphorus tribromide is substituted with a group (HG3) obtained by removing one hydrogen atom from the hydroxy group of the above-mentioned monool, polyalkylene glycol monoether, polyalkylene glycol monoester, polyol, sugar, glycerin fatty acid ester, ethanolamide, or nucleoside (provided that HG1, HG2, and HG3 are different substituents from each other).

### «Step (D1)»

The step (D1) is a step of oxidizing the compound P3 to synthesize an asymmetric phosphoric acid triester. 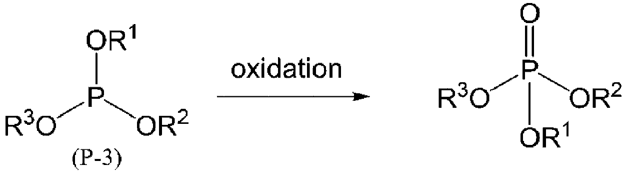 [In the formulae, R¹ to R³ are each independently an organic group. In this regard, R¹ to R³ are all different organic groups.]

The method of oxidizing the compound P3 to obtain an asymmetric phosphoric acid triester is not particularly limited, and for example, the compound P3 can be oxidized by reacting the compound P3 with a specific oxidizing agent.

The oxidizing agent is not particularly limited, and examples thereof include halogen-containing oxidizing agents such as metachloroperbenzoic acid (mCPBA), sodium hypochlorite pentahydrate, trichloroisocyanuric acid, tertiary butyl hypochlorite, N-chlorosuccinimide, N-bromosuccinimide, N-iodosuccinimide, and iodine; hydrogen peroxide solution; and tert-butyl hydroperoxide.

The reaction in the step (A1) to the step (D1) may be carried out in the presence of a solvent. The solvent is not particularly limited, and is preferably a solvent that does not interfere with the reaction of the compound, and preferably a solvent in which the raw materials used in the reaction and the compounds generated in the course of the reaction are highly soluble. Examples of the solvent include dichloromethane (DCM), tetrahydrofuran (THF), diethyl ether (EtzO), 1,4-dioxane, acetonitrile (MeCN), chloroform, and N,N-dimethylformamide (DMF).

The reaction in the step (A1) to the step (D1) may be carried out in the presence of a non-nucleophilic base. The non-nucleophilic base is not particularly limited as long as it can capture HCl generated in the course of the reaction. Examples of the non-nucleophilic base include N,N-diisopropylethylamine (DIEA), triethylamine, diazabicycloundecene, 2,6-lutidine, N-methylmorpholine, N-ethylmorpholine, N-benzyldimethylamine, and tributylamine.

The asymmetric phosphoric acid triester obtained in the step (D1) also includes an asymmetric thiophosphoric acid triester.

For example, an asymmetric thiophosphoric acid triester can be obtained by the reaction as shown below. 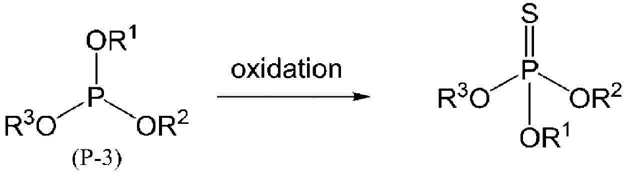 [In the formulae, R¹ to R³ are each independently an organic group. In this regard, R¹ to R³ are all different organic groups.]

The method of oxidizing the compound P3 to obtain an asymmetric thiophosphoric acid triester is not particularly limited, and for example, the compound P3 can be oxidized by reacting the compound P3 with a specific oxidizing agent.

The oxidizing agent is not particularly limited, and examples thereof include xanthane hydride.

The method for producing an asymmetric phosphoric acid triester of the first embodiment described above is a method for producing an asymmetric phosphoric acid triester in which the flow reactor is used, the reaction temperature in the above-mentioned steps (A1) and (B1) is set to -80°C or higher, and the flow rate of each of the phosphorus trichloride or phosphorus tribromide, the first hydroxy compound, and the second hydroxy compound flowing in the flow channel is set to 0.01 mL/min or more. The stirring efficiency can be improved and side reactions can be suppressed by appropriately controlling the reaction temperature and the flow rate.

Therefore, according to the method for producing an asymmetric phosphoric acid triester of the first embodiment, the desired phosphoric acid triester can be produced at a lower cost and with a higher yield.

In the method for producing an asymmetric phosphoric acid triester of the first embodiment described above, the preferred flow rate varies depending on the inner diameter of the mixer of the flow reactor.

For example, the above-mentioned preferred range of the flow rate of each of the phosphorus trichloride or phosphorus tribromide, the first hydroxy compound, and the second hydroxy compound is a particularly preferred range in a case where the inner diameter of the mixer is 0.25 mm.

For example, in a case where the inner diameter of the mixer of the flow reactor is increased by N times (the cross-sectional area is increased by N² times), it is preferable to multiply a preferred value of the each flow rate by N² times.

More specifically, in a case where the inner diameter of the mixer of the flow reactor is 0.25 mm, the flow rate of each compound is preferably 0.01 mL/min or more and 10 mL/min or less, more preferably 0.1 mL/min or more and 8 mL/min or less, still more preferably 1 mL/min or more and 5 mL/min or less, and particularly preferably 2 mL/min or more and 5 mL/min or less.

In a case where the inner diameter of the mixer of the flow reactor is 1 mm, it is preferable to multiply the above value by 4². Specifically, the flow rate of each compound is preferably 0.16 mL/min or more and 160 mL/min or less, more preferably 1.6 mL/min or more and 128 mL/min or less, still more preferably 16 mL/min or more and 80 mL/min or less, and particularly preferably 32 mL/min or more and 80 mL/min or less.

In addition, in the method for producing an asymmetric phosphoric acid triester of the first embodiment described above, it can also be said that, in a case where the inner diameter of the mixer of the flow reactor is defined as ID, the flow rate of each compound is preferably a value obtained by multiplying the above-mentioned preferred flow rate by (ID/0.25)².

That is, in the method for producing an asymmetric phosphoric acid triester of the first embodiment described above, in a case where the inner diameter of the mixer of the flow reactor is defined as ID, the flow rate of each compound is preferably 0.01 × (ID/0.25)² mL/min or more and 10 × (ID/0.25)² mL/min or less, more preferably 0.1 × (ID/0.25)² mL/min or more and 8 × (ID/0.25)² mL/min or less, still more preferably 1 × (ID/0.25)² mL/min or more and 5 × (ID/0.25)² mL/min or less, and particularly preferably 2 × (ID/0.25)² mL/min or more and 5 × (ID/0.25)² mL/min or less.

### <Second embodiment>

The method for producing an asymmetric phosphoric acid triester of the second embodiment is a method for producing an asymmetric phosphoric acid triester in which the asymmetric phosphoric acid triester is synthesized using a flow reactor, the method including step (A1) of reacting phosphorus trichloride or phosphorus tribromide with a first hydroxy compound to synthesize a compound P1, step (N1) of reacting the compound P1 with a base to synthesize a compound N1, step (BN1) of reacting the compound N1 with a second hydroxy compound to synthesize a compound N2, step (CN1) of reacting the compound N2 with a third hydroxy compound to synthesize a compound P3, and step (D1) of oxidizing the compound P3 to synthesize an asymmetric phosphoric acid triester.

The steps (A1) and (D1) in the method for producing an asymmetric phosphoric acid triester of the second embodiment are the same as the steps (A1) and (D1) in the method for producing an asymmetric phosphoric acid triester of the first embodiment described above.

### «Step (N1)»

The step (N1) is a step of reacting the compound P1 with a base (a nucleophilic base) to synthesize a compound N1 represented by the following general formula (N-1). 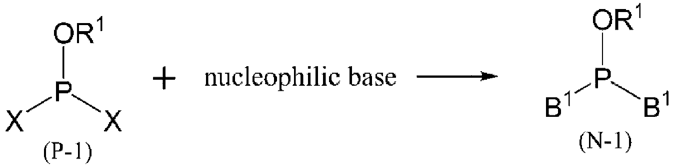 [In the formulae, R¹ is an organic group. B¹ is a group obtained by removing one hydrogen atom from a base. X is a bromine atom or a chlorine atom.]

The base is not particularly limited as long as it is a nucleophilic base.

Specifically, the base is preferably one or more bases selected from the group consisting of pyridine, a pyridine derivative (for example, 4-dimethylaminopyridine (DMAP)), imidazole, an imidazole derivative (for example, N-methylimidazole (NMI) or 4-methylimidazole), and 1,4-diazabicyclo[2,2,2]octane, more preferably imidazole or an imidazole derivative, and still more preferably a compound represented by the following general formula (1-1). 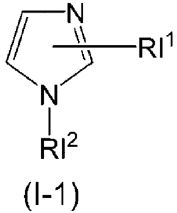 [In the formula, RI¹ and RI² are hydrogen atoms or hydrocarbon groups.]

The hydrocarbon group in RI¹ and RI² in the general formula (1-1) may be an aliphatic hydrocarbon group or an aromatic hydrocarbon group (aryl group).

The aliphatic hydrocarbon group may be linear, branched, or cyclic. In a case of being cyclic, the aliphatic hydrocarbon group may be either monocyclic or polycyclic. The aliphatic hydrocarbon group may have 1 to 20 carbon atoms or 1 to 15 carbon atoms.

The aliphatic hydrocarbon group may be a saturated aliphatic hydrocarbon group (alkyl group) or an unsaturated aliphatic hydrocarbon group, among which an alkyl group is preferable.

The alkyl group may have 1 to 20 carbon atoms, 1 to 10 carbon atoms, or 1 to 5 carbon atoms.

Specific examples of the linear or branched alkyl group include a methyl group, an ethyl group, an n-propyl group, an isopropyl group, an n-butyl group, an isobutyl group, a sec-butyl group, a tert-butyl group, an n-pentyl group, an isopentyl group, a neopentyl group, a tert-pentyl group, a 1-methylbutyl group, an n-hexyl group, a 2-methylpentyl group, a 3-methylpentyl group, a 2,2-dimethylbutyl group, a 2,3-dimethylbutyl group, an n-heptyl group, a 2-methylhexyl group, a 3-methylhexyl group, a 2,2-dimethylpentyl group, a 2,3-dimethylpentyl group, a 2,4-dimethylpentyl group, a 3,3-dimethylpentyl group, a 3-ethylpentyl group, a 2,2,3-trimethylbutyl group, an n-octyl group, an isooctyl group, a nonyl group, a decyl group, an undecyl group, a dodecyl group, a tridecyl group, a tetradecyl group, a pentadecyl group, a hexadecyl group, a heptadecyl group, an octadecyl group, a nonadecyl group, and an icosyl group.

The lower limit value of the reaction temperature in the step (N1) is preferably - 80°C or higher, more preferably -10°C or higher, still more preferably 0°C or higher, particularly preferably 10°C or higher, and most preferably 20°C or higher.

In addition, the upper limit value of the reaction temperature in the step (N1) is not particularly limited, and is, for example, preferably 80°C or lower, more preferably 60°C or lower, and still more preferably 40°C or lower.

In a case where the reaction temperature in the step (N1) is within the above-mentioned preferred range, the reaction proceeds smoothly, and the yield of the compound N1 can be further improved.

For example, the reaction temperature in the step (N1) is preferably -80°C or higher and 80°C or lower, more preferably -10°C or higher and 80°C or lower, still more preferably 0°C or higher and 60°C or lower, particularly preferably 10°C or higher and 40°C or lower, and most preferably 20°C or higher and 40°C or lower.

In the step (N1), the lower limit value of the flow rate of the base flowing in the flow channel is 0.01 mL/min or more, preferably 0.1 mL/min or more, and more preferably 1 mL/min or more.

In addition, in the step (N1), the upper limit value of the flow rate of the base flowing in the flow channel is not particularly limited, and may be, for example, 10 mL/min or less, 8 mL/min or less, or 5 mL/min or less.

In the step (N1), in a case where the flow rate of the base flowing in the flow channel is within the above-mentioned preferred range, the reaction proceeds smoothly, and the yield of the compound N1 can be further improved.

For example, in the step (N1), the flow rate of the base flowing in the flow channel may be 0.01 mL/min or more and 10 mL/min or less, 0.1 mL/min or more and 8 mL/min or less, or 1 mL/min or more and 5 mL/min or less.

In the step (N1), the reaction time for reacting the compound P1 with the base in the mixer of the flow reactor is not particularly limited, and is, for example, 0.01 seconds to 1 minute, and may be 1 to 30 seconds or 1 to 20 seconds.

The compound N1 synthesized in the step (N1)
is a compound in which one chlorine atom of phosphorus trichloride or one bromine atom of phosphorus tribromide is substituted with a group obtained by removing one hydrogen atom from the hydroxy group of R'OH, and the remaining two chlorine atoms of phosphorus trichloride or the remaining two bromine atoms of phosphorus tribromide are both substituted with a group obtained by removing one hydrogen atom from a base.

For example, the compound N1 is a compound in which one chlorine atom of phosphorus trichloride (or one bromine atom of phosphorus tribromide) is substituted with a group obtained by removing one hydrogen atom from the hydroxy group of the above-mentioned monool, polyalkylene glycol monoether, polyalkylene glycol monoester, polyol, sugar, glycerin fatty acid ester, ethanolamide, or nucleoside, and the remaining two chlorine atoms of phosphorus trichloride (or the remaining two bromine atoms of phosphorus tribromide) are substituted with a group obtained by removing one hydrogen atom from pyridine, a pyridine derivative, imidazole, an imidazole derivative, or 1,4-diazabicyclo[2,2,2]octane.

### «Step (BN1)»

The step (BN1) is a step of reacting the compound N1 with a second hydroxy compound to synthesize a compound N2 represented by the following general formula (N-2). 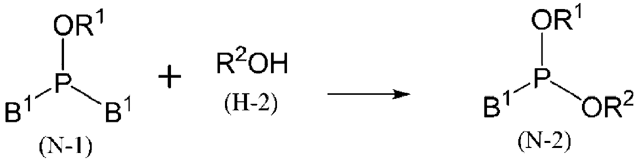 [In the formulae, R¹ and R² are organic groups which are different from each other. B¹ is a group obtained by removing one hydrogen atom from a base.]

The step (BN1) is the same as the step (B1) in the method for producing an asymmetric phosphoric acid triester of the first embodiment described above, except that the compound P1 is the compound N1.

The compound N2 synthesized in the step (BN1) is a compound in which a group obtained by removing one hydrogen atom from one base of the compound N1 is substituted with a group obtained by removing one hydrogen atom from the hydroxy group of R²OH.

For example, the compound N2 is a compound in which one chlorine atom of phosphorus trichloride or one bromine atom of phosphorus tribromide is substituted with a group (HG1) obtained by removing one hydrogen atom from the hydroxy group of the above-mentioned monool, polyalkylene glycol monoether, polyalkylene glycol monoester, polyol, sugar, glycerin fatty acid ester, ethanolamide, or nucleoside, another chlorine atom of phosphorus trichloride or another bromine atom of phosphorus tribromide is substituted with a group (HG2) obtained by removing one hydrogen atom from the hydroxy group of the above-mentioned monool, polyalkylene glycol monoether, polyalkylene glycol monoester, polyol, sugar, glycerin fatty acid ester, ethanolamide, or nucleoside (provided that HG1 and HG2 are substituents different from each other), and the remaining one chlorine atom of phosphorus trichloride or the remaining one bromine atom of phosphorus tribromide is substituted with a group obtained by removing one hydrogen atom from pyridine, a pyridine derivative, imidazole, an imidazole derivative, or 1,4-diazabicyclo[2,2,2]octane.

### <<Step (CN1)>>

The step (CN1) is a step of reacting the compound N2 with a third hydroxy compound to synthesize a compound P3. 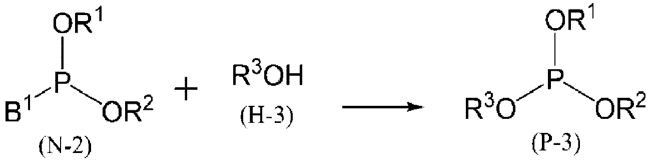 [In the formulae, R¹ to R³ are each independently an organic group. In this regard, R¹ to R³ are all different organic groups. B¹ is a group obtained by removing one hydrogen atom from a base.]

The step (CN1) is the same as the step (C1) in the method for producing an asymmetric phosphoric acid triester of the first embodiment described above, except that the compound P2 is the compound N2.

The reaction in the step (A1), the step (N1), the step (BN1), and the step (CN1) may be carried out in the presence of a solvent. The solvent is not particularly limited, and is preferably a solvent that does not interfere with the reaction of the compound, and preferably a solvent in which the raw materials used in the reaction are highly soluble. Examples of the solvent include dichloromethane (DCM), tetrahydrofuran (THF), diethyl ether (EtzO), 1,4-dioxane, acetonitrile (MeCN), chloroform, and N,N-dimethylformamide (DMF).

The reaction in the step (A1), the step (N1), the step (BN1), and the step (CN1) may be carried out in the presence of a non-nucleophilic base. The non-nucleophilic base is not particularly limited as long as it can capture HCl generated in the course of the reaction. Examples of the non-nucleophilic base include N,N-diisopropylethylamine (DIEA), triethylamine, diazabicycloundecene, 2,6-lutidine, N-methylmorpholine, N-ethylmorpholine, N-benzyldimethylamine, and tributylamine.

The method for producing an asymmetric phosphoric acid triester of the second embodiment described above further includes the step (N1) in addition to each step of the method for producing an asymmetric phosphoric acid triester of the first embodiment. The reactivity can be appropriately suppressed by substituting each of the two chlorine atoms of the compound P1 (or the two bromine atoms of the compound P1) with a group obtained by removing one hydrogen atom from a base.

Therefore, according to the method for producing an asymmetric phosphoric acid triester of the second embodiment, the desired phosphoric acid triester can be produced with a higher yield.

Also in the method for producing an asymmetric phosphoric acid triester of the second embodiment, the relationship between the preferred flow rate and the inner diameter of the mixer of the flow reactor is the same as that of the method for producing an asymmetric phosphoric acid triester of the first embodiment described above.

That is, in a case where the inner diameter of the mixer of the flow reactor is defined as ID, the flow rate of each compound is preferably a value obtained by multiplying the above-mentioned preferred flow rate by (ID/0.25)².

### (Method for producing symmetric phosphoric acid triester according to second aspect of present disclosure)

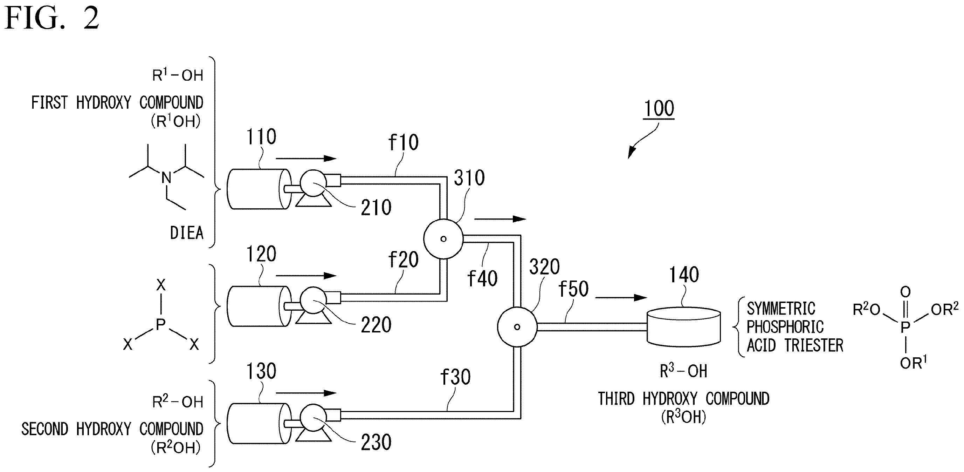

Hereinafter, the form of a flow reactor according to the present embodiment and the method for producing a symmetric phosphoric acid triester using the flow reactor will be described with reference to FIG. 2.

FIG. 2 is a schematic diagram showing a schematic configuration of a flow reactor 100. The flow reactor 100 includes a tank 110 for accommodating a first liquid, a tank 120 for accommodating a second liquid, a tank 130 for accommodating a third liquid, and a tank 140 for accommodating a mixed liquid of the third liquid and a fourth liquid.

For example, the first liquid contains a first hydroxy compound (R¹OH) and N,N-diisopropylethylamine (DIEA), the second liquid contains phosphorus trichloride or phosphorus tribromide, and the third liquid contains a second hydroxy compound (R²OH).

The flow reactor 100 includes flow channels f10, f20, f30, f40, and f50 for transporting fluids. The inner diameters of the flow channel and the mixer may be, for example, 0.1 to 1 mm, 0.1 to 0.8 mm, or 0.2 to 0.6 mm. In addition, the inner diameters of the flow channel and the mixer may be 1 mm to several cm.

The tanks 110, 120, 130, and 140, and the flow channels f10, f20, f30, f40, and f50 are formed of, for example, resins such as plastics or elastomers, glass materials, metals, or ceramics.

The tank 110 is connected to a pump 210, and the operation of the pump 210 causes the first liquid accommodated in the tank 110 to move in the flow channel f10 and flow into a mixer 310. The tank 120 is connected to a pump 220, and the operation of the pump 220 causes the second liquid accommodated in the tank 120 to move in the flow channel f20 and flow into the mixer 310. Next, the first liquid and the second liquid are mixed by the mixer 310 to form a first mixed liquid, which is sent to the flow channel f50. In this mixing process, R¹OH contained in the first liquid reacts with phosphorus trichloride or phosphorus tribromide contained in the second liquid to obtain a compound P1 in which one chlorine atom of phosphorus trichloride or one bromine atom of phosphorus tribromide is substituted with an R¹O- group (step A2 of the method for producing a symmetric phosphoric acid triester). The first mixed liquid containing the obtained compound P1 moves in the flow channel f40 and flows into a mixer 320.

The tank 130 is connected to a pump 230, and the operation of the pump 230 causes the third liquid accommodated in the tank 130 to move in the flow channel f30 and flow into the mixer 320. Next, the third liquid and the first mixed liquid are mixed to form a second mixed liquid, which is sent to the flow channel f50. In this mixing process, the compound P1 obtained in the step A2 reacts with R²OH contained in the third liquid to obtain a compound P4 in which two chlorine atoms of the compound P1 (or two bromine atoms of the compound P1) are each substituted with an R²O- group (step 82 of the method for producing a symmetric phosphoric acid triester). The obtained compound P4 moves in the flow channel f50 and is stored in the tank 140. Subsequently, a symmetric phosphoric acid triester is synthesized by oxidizing the compound P4, and the symmetric phosphoric acid triester is stored in the tank 140 (step D2 of the method for producing a symmetric phosphoric acid triester).

The compound P1 is highly reactive, and a side reaction in which the compound P1 reacts again with R'OH is likely to occur, so that it is important to control the reaction.

According to the flow reactor 100 according to the present embodiment, the synthesized compound P1 can be rapidly released from the mixer, and thus the side reactions can be suppressed, making it possible to produce the desired phosphoric acid triester with high yield.

The flow reactor 100 has been exemplified as a form in which the liquids are mixed by a mixer, but the mixing of the liquids can be achieved only by communication between the flow channels, so the flow reactor of the present embodiment does not necessarily have a mixer.

### <Third embodiment>

The method for producing a symmetric phosphoric acid triester according to the third embodiment is a method for producing a symmetric phosphoric acid triester in which the symmetric phosphoric acid triester is synthesized using a flow reactor, the method including step (A2) of reacting phosphorus trichloride or phosphorus tribromide with a first hydroxy compound (R'OH) to synthesize a compound P1 (hereinafter, referred to as "step (A2)"), step (B2) of reacting the compound P1 with a second hydroxy compound (R²OH) to synthesize a compound P4 (hereinafter, referred to as "step (B2)"), and step (D2) of oxidizing the compound P4 to synthesize a symmetric phosphoric acid triester (hereinafter, referred to as "step (D2)"). 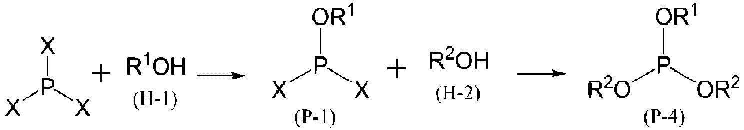 [In the formulae, R¹ and R² are organic groups different from each other. X is a bromine atom or a chlorine atom.]

The reaction temperature in the steps (A2) and (B2) is -80°C or higher, and in the steps (A2) and (B2), the flow rate of each of the phosphorus trichloride or phosphorus tribromide, the first hydroxy compound, and the second hydroxy compound flowing in the flow channel is 0.01 mL/min or more.

In addition, the preferred ranges of the reaction temperature and the flow rate are the same as the ranges described in the step (A1) and the step (B1) in the method for producing an asymmetric phosphoric acid triester of the first embodiment described above.

In addition, the step (D2) is also the same as the content described in the step (D1) in the method for producing an asymmetric phosphoric acid triester of the first embodiment described above.

The reaction in the steps (A2) and (B2) may be carried out in the presence of a solvent. The solvent is not particularly limited, and is preferably a solvent that does not interfere with the reaction of the compound, and preferably a solvent in which the raw materials used in the reaction are highly soluble. Examples of the solvent include dichloromethane (DCM), tetrahydrofuran (THF), diethyl ether (EtaO), 1,4-dioxane, acetonitrile (MeCN), chloroform, and N,N-dimethylformamide (DMF).

The reaction in the steps (A2) and (B2) may be carried out in the presence of a non-nucleophilic base. The non-nucleophilic base is not particularly limited as long as it can capture HCl generated in the course of the reaction. Examples of the non-nucleophilic base include N,N-diisopropylethylamine (DIEA), triethylamine, diazabicycloundecene, 2,6-lutidine, N-methylmorpholine, N-ethylmorpholine, N-benzyldimethylamine, and tributylamine.

The method for producing an asymmetric phosphoric acid triester of the third embodiment described above is a method for producing a symmetric phosphoric acid triester in which the flow reactor is used, the reaction temperature in the above-mentioned steps (A2) and (B2) is set to -80°C or higher, and the flow rate of each of the phosphorus trichloride or phosphorus tribromide, the first hydroxy compound, and the second hydroxy compound flowing in the flow channel is set to 0.01 mL/min or more. The stirring efficiency can be improved and side reactions can be suppressed by appropriately controlling the reaction temperature and the flow rate.

Therefore, according to the method for producing a symmetric phosphoric acid triester of the third embodiment, the desired phosphoric acid triester can be produced at a lower cost and with a higher yield.

Also in the method for producing an asymmetric phosphoric acid triester of the third embodiment, the relationship between the preferred flow rate and the inner diameter of the mixer of the flow reactor is the same as that of the method for producing an asymmetric phosphoric acid triester of the first embodiment described above.

That is, in a case where the inner diameter of the mixer of the flow reactor is defined as ID, the flow rate of each compound is preferably a value obtained by multiplying the above-mentioned preferred flow rate by (ID/0.25)².

### (Method for producing phosphoric acid ester according to third aspect of present disclosure)

The method for producing a phosphoric acid ester according to the third aspect of the present disclosure is a method for producing a phosphoric acid ester in which the phosphoric acid ester is synthesized using a flow reactor, the method including step (A11) of reacting phosphorus trichloride or phosphorus tribromide with a first hydroxy compound represented by general formula (H1-1) below to synthesize a compound P11 represented by general formula (P1-1) below (hereinafter, referred to as "step (A11)"), step (B11) of reacting the compound P11 with a second hydroxy compound represented by general formula (H1-2 below) to synthesize a compound P12 represented by general formula (P1-2) below (hereinafter, referred to as "step (81 1)"), step (C11) of reacting the compound P12 with a third hydroxy compound represented by general formula (H1-3) below to synthesize a compound P13 represented by general formula (P1-3) below (hereinafter, referred to as "step (C11)"), and step (D11) of oxidizing the compound P13 to synthesize a phosphoric acid ester (hereinafter, referred to as "step (D11)").

Unlike the production methods of the foregoing aspects, in the method for producing a phosphoric acid ester according to the third aspect of the present disclosure, at least one of the first to third hydroxy compounds is water, so the method for producing a phosphoric acid ester is specifically a method for producing a phosphoric acid monoester or a phosphoric acid diester. 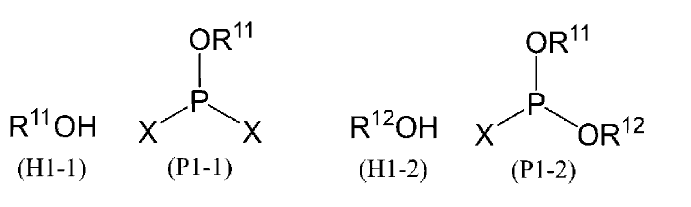 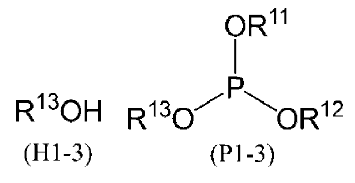 [R¹¹ to R¹³ are each independently a hydrogen atom or an organic group. In this regard, at least one of R¹¹ to R¹³ is an organic group, and at least one of R¹¹ to R¹³ is a hydrogen atom. X is a bromine atom or a chlorine atom.]

An example of the reaction formula in a case where the method for producing a phosphoric acid ester is a method for producing a phosphoric acid monoester is shown below. In reaction formula below, the compound P12 represented by the general formula (P1-2) in a case where the second hydroxy compound is water is denoted as a compound P12-1, and the compound P13 represented by the general formula (P1-3) in a case where the second hydroxy compound is water and the third hydroxy compound is also water is denoted as a compound P13-1.

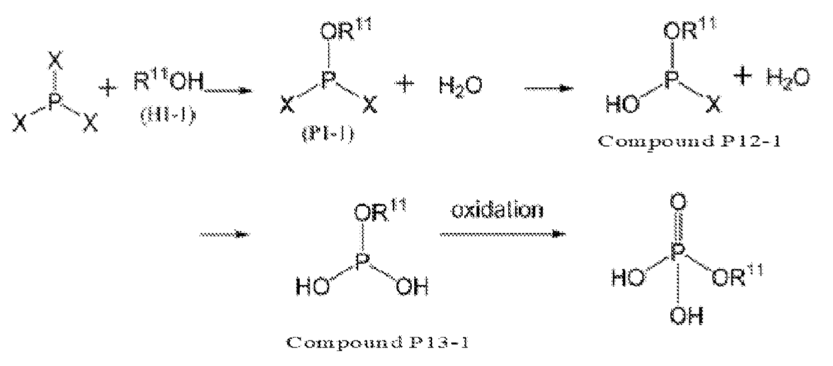

An example of the reaction formula in a case where the method for producing a phosphoric acid ester is a method for producing a phosphoric acid diester is shown below. In reaction formula below, the compound P13 represented by the general formula (P1-3) in a case where only the third hydroxy compound is water is denoted as a compound P13-2.

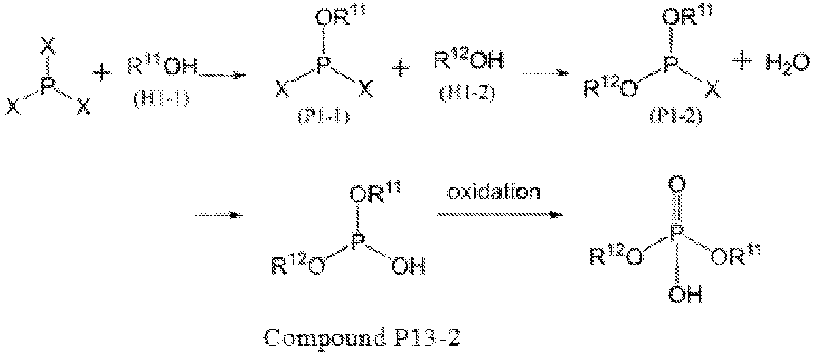

The first hydroxy compound, the second hydroxy compound, and the third hydroxy compound in the method for producing a phosphoric acid ester according to the third aspect of the present disclosure include the same hydroxy compounds as exemplified for R¹OH, R²OH, and R³OH described above, and water.

The reaction temperature in the steps (A11) and (B11) is -80°C or higher, and in the steps (A11) and (B11), the flow rate of each of the phosphorus trichloride or phosphorus tribromide, the first hydroxy compound, and the second hydroxy compound flowing in the flow channel is 0.01 mL/min or more.

In addition, the preferred ranges of the reaction temperature and the flow rate are the same as the ranges described in the step (A1) and the step (B1) in the method for producing an asymmetric phosphoric acid triester of the first embodiment described above.

In addition, the step (D11) is also the same as the content described in the step (D1) in the method for producing an asymmetric phosphoric acid triester of the first embodiment described above.

The reaction in the steps (A11) and (B11) may be carried out in the presence of a solvent. The solvent is not particularly limited, and is preferably a solvent that does not interfere with the reaction of the compound, and preferably a solvent in which the raw materials used in the reaction are highly soluble. Examples of the solvent include dichloromethane (DCM), tetrahydrofuran (THF), diethyl ether (Et₂O), 1,4-dioxane, acetonitrile (MeCN), chloroform, and N,N-dimethylformamide (DMF).

The reaction in the steps (A1 1) and (B11) may be carried out in the presence of a non-nucleophilic base. The non-nucleophilic base is not particularly limited as long as it can capture HCl generated in the course of the reaction. Examples of the non-nucleophilic base include N,N-diisopropylethylamine (DIEA), triethylamine, diazabicycloundecene, 2,6-lutidine, N-methylmorpholine, N-ethylmorpholine, N-benzyldimethylamine, and tributylamine.

The method for producing a phosphoric acid ester according to the third aspect of the present disclosure described above is a method for producing a phosphoric acid monoester or a phosphoric acid diester in which the flow reactor is used, the reaction temperature in the above-mentioned steps (A11) and (B11) is set to -80°C or higher, and the flow rate of each of the phosphorus trichloride or phosphorus tribromide, the first hydroxy compound, and the second hydroxy compound flowing in the flow channel is set to 0.01 mL/min or more. The stirring efficiency can be improved and side reactions can be suppressed by appropriately controlling the reaction temperature and the flow rate.

Therefore, according to the method for producing a phosphoric acid ester according to the third aspect of the present disclosure, the desired phosphoric acid monoester or phosphoric acid diester can be produced at a lower cost and with a higher yield.

Also in the method for producing a phosphoric acid ester according to the third aspect of the present disclosure, the relationship between the preferred flow rate and the inner diameter of the mixer of the flow reactor is the same as that of the method for producing an asymmetric phosphoric acid triester of the first embodiment described above.

That is, in a case where the inner diameter of the mixer of the flow reactor is defined as ID, the flow rate of each compound is preferably a value obtained by multiplying the above-mentioned preferred flow rate by (ID/0.25)².

### (Method for producing organophosphorus compound according to fourth aspect of present disclosure)

A method for producing an organophosphorus compound according to a fourth aspect of the present disclosure is a method for producing an organophosphorus compound in which the organophosphorus compound is synthesized using a flow reactor, the method including step (A01) of reacting phosphorus trichloride or phosphorus tribromide with a first hydroxy compound represented by general formula (H0-1) below to synthesize a compound P01 represented by general formula (P0-1) below, step (B01) of reacting the compound P01 with a second hydroxy compound represented by general formula (H0-2) below to synthesize a compound P02 represented by general formula (P0-2) below, and step (C01) of reacting the compound P02 with a third hydroxy compound to synthesize a compound P03 represented by general formula (P0-3) below, in which the reaction temperature in the steps (A01) and (B01) is -80°C or higher, and in the steps (A01) and (B01), the flow rate of each of the phosphorus trichloride or phosphorus tribromide, the first hydroxy compound, and the second hydroxy compound flowing in a flow channel is 0.01 mL/min or more. 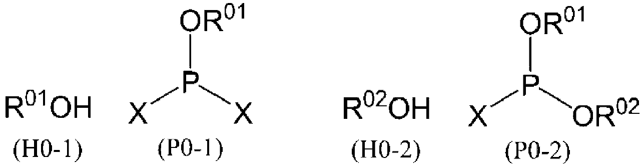 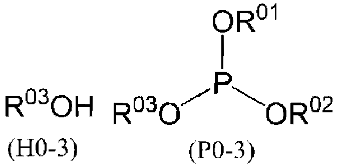 [R⁰¹ to R⁰³ are each independently a hydrogen atom or an organic group. In this regard, at least one of R⁰¹ to R⁰³ is an organic group, and in a case where all of R⁰¹ to R⁰³ are organic groups, not all three of the organic groups are the same. X is a bromine atom or a chlorine atom.]

More specifically, the method for producing an organophosphorus compound according to the fourth aspect of the present disclosure is a method for producing an intermediate in the method for producing an asymmetric phosphoric acid triester according to the first aspect of the present disclosure, the method for producing a symmetric phosphoric acid triester according to the second aspect of the present disclosure, and the method for producing a phosphoric acid ester according to the third aspect of the present disclosure. That is, the compound P03 is a compound containing all of the compound P3, the compound P4, and the compound P13 described above.

The organophosphorus compound also includes a tautomer.

Examples of the tautomer of the above-mentioned compound P13 include tautomers of the compound P13-1 and the compound P13-2 described above, specific examples of which include a compound P13-1-1 and a compound P13-2-1 shown below.

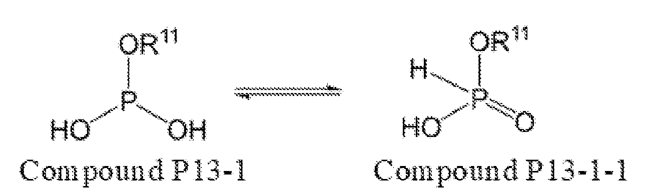

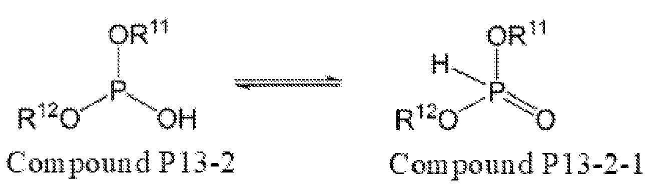

Specific examples of the first hydroxy compound, the second hydroxy compound, and the third hydroxy compound in the method for producing an organophosphorus compound according to the fourth aspect of the present disclosure include the same hydroxy compounds as exemplified for R'OH, R²OH, and R³OH described above, and water.

The reaction temperature in the steps (A01) and (B01) is -80°C or higher, and in the steps (A01) and (B01), the flow rate of each of the phosphorus trichloride or phosphorus tribromide, the first hydroxy compound, and the second hydroxy compound flowing in the flow channel is 0.01 mL/min or more.

In addition, the preferred ranges of the reaction temperature and the flow rate are the same as the ranges described in the step (A1) and the step (B1) in the method for producing an asymmetric phosphoric acid triester of the first embodiment described above.

The reaction in the steps (A01) and (B01) may be carried out in the presence of a solvent. The solvent is not particularly limited, and is preferably a solvent that does not interfere with the reaction of the compound, and preferably a solvent in which the raw materials used in the reaction are highly soluble. Examples of the solvent include dichloromethane (DCM), tetrahydrofuran (THF), diethyl ether (Et₂O), 1,4-dioxane, acetonitrile (MeCN), chloroform, and N,N-dimethylformamide (DMF).

The reaction in the steps (A01) and (B01) may be carried out in the presence of a non-nucleophilic base. The non-nucleophilic base is not particularly limited as long as it can capture HCl generated in the course of the reaction. Examples of the non-nucleophilic base include N,N-diisopropylethylamine (DIEA), triethylamine, diazabicycloundecene, 2,6-lutidine, N-methylmorpholine, N-ethylmorpholine, N-benzyldimethylamine, and tributylamine.

The method for producing an organophosphorus compound according to the fourth aspect of the present disclosure described above is a method for producing an organophosphorus compound (intermediate) used for producing a phosphoric acid monoester, a phosphoric acid diester, or a phosphoric acid triester, in which the flow reactor is used, the reaction temperature in the above-mentioned steps (A01) and (B01) is set to -80°C or higher, and the flow rate of each of the phosphorus trichloride or phosphorus tribromide, the first hydroxy compound, and the second hydroxy compound flowing in the flow channel is set to 0.01 mL/min or more. The stirring efficiency can be improved and side reactions can be suppressed by appropriately controlling the reaction temperature and the flow rate.

Therefore, according to the organophosphorus compound according to the fourth aspect of the present disclosure, an organophosphorus compound (intermediate) used for producing the desired phosphoric acid monoester, phosphoric acid diester, or phosphoric acid triester can be produced at a lower cost and with a higher yield.

Also in the method for producing an organophosphorus compound according to the fourth aspect of the present disclosure, the relationship between the preferred flow rate and the inner diameter of the mixer of the flow reactor is the same as that of the method for producing an asymmetric phosphoric acid triester of the first embodiment described above.

That is, in a case where the inner diameter of the mixer of the flow reactor is defined as ID, the flow rate of each compound is preferably a value obtained by multiplying the above-mentioned preferred flow rate by (ID/0.25)².

### (Method for producing organophosphorus compound according to fifth aspect of present disclosure)

A method for producing an organophosphorus compound according to a fifth aspect of the present disclosure is a method for producing an organophosphorus compound in which the organophosphorus compound is synthesized using a flow reactor, the method including step (AZ1) of reacting phosphorus trichloride or phosphorus tribromide with a first compound which is any of a hydroxy compound, an amine compound, or a thiol compound to synthesize a compound PZ1 represented by general formula (PZ-1) below, step (BZ1) of reacting the compound PZ1 with a second compound which is any of a hydroxy compound, an amine compound, or a thiol compound to synthesize a compound PZ2 represented by general formula (PZ-2) below, and step (CZ1) of reacting the compound PX2 with a third compound which is any of a hydroxy compound, an amine compound, or a thiol compound to synthesize a compound PZ3 represented by general formula (PZ-3) below, in which the reaction temperature in the steps (AZ1) and (BZ1) is -80°C or higher, and in the steps (AZ1) and (BZ1), the flow rate of each of the phosphorus trichloride or phosphorus tribromide, the first compound, and the second compound flowing in a flow channel is 0.01 mL/min or more. 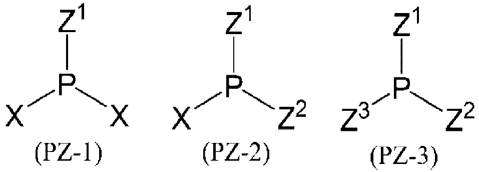 [In the formulae, Z¹ to Z³ are each independently a hydrogen atom, a group obtained by removing one hydrogen atom from a hydroxy compound, a group obtained by removing one hydrogen atom from an amine compound, or a group obtained by removing one hydrogen atom from a thiol compound. In this regard, not all three of Z¹ to Z³ are the same. X is a bromine atom or a chlorine atom.]

The reaction temperature in the steps (AZ1) and (BZ1) is -80°C or higher, and in the steps (AZ1) and (BZ1), the flow rate of each of the phosphorus trichloride or phosphorus tribromide, the first compound, and the second compound flowing in the flow channel is 0.01 mL/min or more.

In addition, the preferred ranges of the reaction temperature and the flow rate are the same as the ranges described in the step (A1) and the step (B1) in the method for producing an asymmetric phosphoric acid triester of the first embodiment described above.

The reaction in the steps (AZ1) and (BZ1) may be carried out in the presence of a solvent. The solvent is not particularly limited, and is preferably a solvent that does not interfere with the reaction of the compound, and preferably a solvent in which the raw materials used in the reaction are highly soluble. Examples of the solvent include dichloromethane (DCM), tetrahydrofuran (THF), diethyl ether (Et₂O), 1,4-dioxane, acetonitrile (MeCN), chloroform, and N,N-dimethylformamide (DMF).

The reaction in the steps (AZ1) and (BZ1) may be carried out in the presence of a non-nucleophilic base. The non-nucleophilic base is not particularly limited as long as it can capture HCl generated in the course of the reaction. Examples of the non-nucleophilic base include N,N-diisopropylethylamine (DIEA), triethylamine, diazabicycloundecene, 2,6-lutidine, N-methylmorpholine, N-ethylmorpholine, N-benzyldimethylamine, and tributylamine.

### <First to third compounds>

The first to third compounds are different hydroxy compounds, amine compounds, or thiol compounds which are different from each other.

### <Hydroxy compound>

Examples of the hydroxy compounds in the first to third compounds include the same hydroxy compounds as exemplified for R¹OH, R²OH, and R³OH described above, and water.

### <Amine compound>

Examples of the amine compounds in the first to third compounds include an aliphatic amine, a composite amine, an aromatic amine, a heterocyclic amine, a nitrogen-containing compound having a carboxy group, a nitrogen-containing compound having a sulfonyl group, a nitrogen-containing compound having a hydroxyl group, an amide derivative, and an imide derivative.

Examples of the aliphatic amine include an aliphatic primary amine and an aliphatic secondary amine.

Specific examples of the aliphatic primary amine include methylamine, ethylamine, n-propylamine, isopropylamine, n-butylamine, isobutylamine, sec-butylamine, tert-butylamine, pentylamine, tert-amylamine, cyclopentylamine, hexylamine, cyclohexylamine, heptylamine, octylamine, nonylamine, decylamine, dodecylamine, cetylamine, methylenediamine, ethylenediamine, and tetraethylenepentamine.

Specific examples of the aliphatic secondary amine include dimethylamine, diethylamine, di-n-propylamine, diisopropylamine, di-n-butylamine, diisobutylamine, di-sec-butylamine, dipentylamine, dicyclopentylamine, dihexylamine, dicyclohexylamine, diheptylamine, dioctylamine, dinonylamine, didecylamine, didodecylamine, dicetylamine, N,N-dimethylmethylenediamine, N,N-dimethylethylenediamine, and N,N-dimethyltetraethylenepentamine.

Specific examples of the composite amine include benzylamine, phenethylamine, and benzyldimethylamine.

Specific examples of the aromatic amine and the heterocyclic amine include an aniline derivative (for example, aniline, N-methylaniline, N-ethylaniline, N-propylaniline, 2-methylaniline, 3-methylaniline, 4-methylaniline, ethylaniline, propylaniline, trimethylaniline, 2-nitroaniline, 3-nitroaniline, 4-nitroaniline, 2,4-dinitroaniline, 2,6-dinitroaniline, or 3,5-dinitroaniline), diphenyl(p-tolyl)amine, phenylenediamine, naphthylamine, diaminonaphthalene, a pyrrole derivative (for example, pyrrole, 1-methylpyrrole, 2,4-dimethylpyrrole, or 2,5-dimethylpyrrole), an imidazole derivative (for example, imidazole, 4-methylimidazole, or 4-methyl-2-phenylimidazole), a pyrazole derivative, a pyrroline derivative (for example, 2-pyrroline or 3-pyrroline), a pyrrolidine derivative (for example, 2-methyl-pyrrolidine), an imidazoline derivative, an imidazolidine derivative, a pyrazoline derivative, a pyrazolidine derivative, a piperidine derivative, a piperazine derivative, a morpholine derivative, an indole derivative, an isoindole derivative, a 1 H-indazole derivative, an indoline derivative, a purine derivative, a carbazole derivative, an adenine derivative, an adenosine derivative, a guanine derivative, a guanosine derivative, an uracil derivative, and a uridine derivative.

Specific examples of the nitrogen-containing compound having a carboxy group include aminobenzoic acid, indole carboxylic acid, and an amino acid derivative (for example, nicotinic acid, alanine, arginine, aspartic acid, glutamic acid, glycine, histidine, isoleucine, glycylleucine, leucine, methionine, phenylalanine, threonine, lysine, 3-aminopyrazine-2-carboxylic acid, or methoxyalanine).

Specific examples of the nitrogen-containing compound having a hydroxyl group include aminocresol, 3-indole methanol hydrate, monoethanolamine, diethanolamine, 2,2'-iminodiethanol, 2-aminoethanol, 3-amino-1-propanol, 4-amino-1-butanol, 1-(2-hydroxyethyl)piperazine, 1-[2-(2-hydroxyethoxy)ethyl]piperazine, and piperidineethanol.

Specific examples of the amide derivative include formamide, N-methylformamide, acetamide, N-methylacetamide, propionamide, and benzamide.

Specific examples of the imide derivative include phthalimide, succinimide, and maleimide.

### <Thiol compound>

Examples of the thiol compounds in the first to third compounds include an aliphatic thiol compound, an aromatic thiol compound, an aliphatic polythiol compound, a mercaptocarboxylic acid ester compound, mercaptocarboxylic acid, and mercaptoether.

Specific examples of the aliphatic thiol compound include methylthiol, ethylthiol, 1-propylthiol (n-propyl mercaptan), isopropylthiol, 1-butylthiol (n-butyl mercaptan), isobutylthiol, tert-butylthiol, 1-pentylthiol, isopentylthiol, 2-pentylthiol, 3-pentylthiol, 1-hexylthiol, cyclohexylthiol, 4-methyl-2-pentylthiol, 1-heptylthiol (n-heptyl mercaptan), 2-heptylthiol, 1-octylthiol (n-octyl mercaptan), isooctylthiol, 2-ethylhexylthiol, 2,4,4-trimethyl-2-pentylthiol (tert-octyl mercaptan), 1-nonylthiol (n-nonyl mercaptan), tert-nonylthiol (tert-nonyl mercaptan), isononylthiol, 1-decylthiol, 1-undecylthiol, 1-dodecylthiol, tert-dodecylthiol (tert-dodecyl mercaptan), tridecylthiol, tetradecylthiol, 1-pentyldecylthiol, 1-hexyldecylthiol, 1-heptyldecylthiol, 1-octyl decyl thiol, nonadecylthiol, eicosanethiol, triacontanethiol, tetracontanethiol, pentacontanethiol, hexacontanethiol, heptacontanethiol, octacontanethiol, nonacontanethiol, hectanethiol, 1-myristylthiol, cetylthiol, 1-stearylthiol, isostearylthiol, 2-octyldecylthiol, 2-octyldodecylthiol, 2-hexyldecylthiol, and behenylthiol.

Specific examples of the aromatic thiol compound include benzenethiol, phenylmethanethiol, xylenethiol, 1,2-dimercaptobenzene, 1,3-dimercaptobenzene, 1,4-dimercaptobenzene, 1,2-bis(mercaptomethyl)benzene, 1,3-bis(mercaptomethyl)benzene, 1,4-bis(mercaptomethyl)benzene, 1,2-bis(2-mercaptoethyl)benzene, 1,3-bis(2-mercaptoethyl)benzene, 1,4-bis(2-mercaptoethyl)benzene, 1,2-bis(2-mercaptoethyleneoxy)benzene, 1,3-bis(2-mercaptoethyleneoxy)benzene, 1,4-bis(2-mercaptoethyleneoxy)benzene, 1,2,3-trimercaptobenzene, 1,2,4-trimercaptobenzene, 1,3,5-trimercaptobenzene, 1,2,3-tris(mercaptomethyl)benzene, 1,2,4-tris(mercaptomethyl)benzene, 1,3,5-tris(mercaptomethyl)benzene, 1,2,3-tris(2-mercaptoethyl)benzene, 1,2,4-tris(2-mercaptoethyl)benzene, 1,3,5-tris(2-mercaptoethyl)benzene, 1,2,3-tris(2-mercaptoethyleneoxy)benzene, 1,2,4-tris(2-mercaptoethyleneoxy)benzene, 1,3,5-tris(2-mercaptoethyleneoxy)benzene, 1,2,3,4-tetramercaptobenzene, 1,2,3,5-tetramercaptobenzene, 1,2,4,5-tetramercaptobenzene, 1,2,3,4-tetrakis(mercaptomethyl)benzene, 1,2,3,5-tetrakis(mercaptomethyl)benzene, 1,2,4,5-tetrakis(mercaptomethyl)benzene, 1,2,3,4-tetrakis(2-mercaptoethyl)benzene, 1,2,3,5-tetrakis(2-mercaptoethyl)benzene, 1,2,4,5-tetrakis(2-mercaptoethyl)benzene, 1,2,3,4-tetrakis(2-mercaptoethyleneoxy)benzene, 1,2,3,5-tetrakis(2-mercaptoethyleneoxy)benzene, 1,2,4,5-tetrakis(2-mercaptoethyleneoxy)benzene, 2,2'-dimercaptobiphenyl, 4,4'-thiobis-benzenethiol, 4,4'-dimercaptobiphenyl, 4,4'-dimercaptobibenzyl, 2,5-toluenedithiol, 3,4-toluenedithiol, 1,4-naphthalenedithiol, 1,5-naphthalenedithiol, 2,6-naphthalenedithiol, 2,7-naphthalenedithiol, 2,4-dimethylbenzene-1,3-dithiol, 4,5-dimethylbenzene-1,3-dithiol, 9,10-anthracenedimethanethiol, 1,3-bis(2-mercaptoethylthio)benzene, 1,4-bis(2-mercaptoethylthio)benzene, 1,2-bis(2-mercaptoethylthiomethyl)benzene, 1,3-bis(2-mercaptoethylthiomethyl)benzene, 1,4-bis(2-mercaptoethylthiomethyl)benzene, 1,2,3-tris(2-mercaptoethylthio)benzene, 1,2,4-tris(2-mercaptoethylthio)benzene, 1,3,5-tris(2-mercaptoethylthio)benzene, 1,2,3,4-tetrakis(2-mercaptoethylthio)benzene, 1,2,3,5-tetrakis(2-mercaptoethylthio)benzene, 1,2,4,5-tetrakis(2-mercaptoethylthio)benzene, benzylthiol, m-toluenethiol, p-toluenethiol, 2-naphthalenethiol, 2-pyridylthiol, 2-mercaptobenzoimidazole, and 2-mercaptobenzothiazole.

Examples of the aliphatic polythiol compound include dithiol compounds such as methanedithiol, 1,2-ethanedithiol, 1,2-propanedithiol, 1,3-propanedithiol, 1,4-butanedithiol, 1,6-hexanedithiol, 1,7-heptanedithiol, 1,8-octanedithiol, 1,9-nonanedithiol, 1,10-decanedithiol, 1,12-dodecanedithiol, 2,2-dimethyl-1,3-propanedithiol, 3-methyl-1,5-pentanedithiol, 2-methyl-1,8-octanedithiol, 1,4-cyclohexanedithiol, 1,4-bis(mercaptomethyl)cyclohexane, 1,1-cyclohexanedithiol, 1,2-cyclohexanedithiol, bicyclo[2,2,1]hepta-exo-cis-2,3-dithiol, 1,1-bis(mercaptomethyl)cyclohexane, bis(2-mercaptoethyl)ether, ethylene glycol bis(2-mercaptoacetate), and ethylene glycol bis(3-mercaptopropionate); trithiol compounds such as 1,1,1-tris(mercaptomethyl)ethane, 2-ethyl-2-mercaptomethyl-1,3-propanedithiol, 1,2,3-propanetrithiol, trimethylolpropane tris(2-mercaptoacetate), trimethylolpropane tris(3-mercaptopropionate), and tris((mercaptopropionyloxy)-ethyl)isocyanurate; and thiol compounds having four or more SH groups such as pentaerythritol tetrakis(2-mercaptoacetate), pentaerythritol tetrakis(3-mercaptopropionate), pentaerythritol tetrakis(3-mercaptobutanate), and dipentaerythritol hexa-3-mercaptopropionate.

Examples of the mercaptocarboxylic acid ester compound include methyl mercaptoacetate, methyl 3-mercaptopropionate, 4-methoxybutyl 3-mercaptopropionate, 2-ethylhexyl 3-mercaptopropionate, n-octyl 3-mercaptopropionate, stearyl 3-mercaptopropionate, 1,4-bis(3-mercaptopropionyloxy)butane, 1,4-bis(3-mercaptobutyryloxy)butane, trimethylolethane tris(3-mercaptopropionate), trimethylolethane tris(3-mercaptobutyrate), trimethylolpropane tris(3-mercaptopropionate), trimethylolpropane tris(3-mercaptobutyrate), pentaerythritol tetrakis(3-mercaptopropionate), pentaerythritol tetrakis(3-mercaptobutyrate), dipentaerythritol hexakis(3-mercaptopropionate), dipentaerythritol hexakis(3-mercaptobutyrate), tris[2-(3-mercaptopropionyloxy)ethyl]isocyanurate, and tris[2-(3-mercaptobutyryloxy)ethyl]isocyanurate.

Examples of other thiol compounds include mercaptocarboxylic acids such as mercaptoacetic acid, 3-mercaptopropionic acid, 2-mercaptopropionic acid, 3-mercaptobutyric acid, mercaptohexanoic acid, mercaptooctanoic acid, mercaptostearic acid, and thioglycolic acid; mercaptoethers such as di(mercaptoethyl)ether; mercapto alcohol compounds such as 2-mercaptoethanol and 4-mercapto-1-butanol; and silane-containing thiol compounds such as (y-mercaptopropyl)trimethoxysilane and (γ-mercaptopropyl)triethoxysilane.

In the formulae (PZ-1) to (PZ-3), Z¹ to Z³ are each independently a hydrogen atom, a group obtained by removing one hydrogen atom from the above-mentioned hydroxy compound, a group obtained by removing one hydrogen atom from the above-mentioned amine compound, or a group obtained by removing one hydrogen atom from the above-mentioned thiol compound. In this regard, not all three of Z¹ to Z³ are the same.

The method for producing an organophosphorus compound according to the fifth aspect of the present disclosure described above is a method for producing an organophosphorus compound in which the flow reactor is used, the reaction temperature in the above-mentioned steps (AZ1) and (BZ1) is set to -80°C or higher, and the flow rate of each of the phosphorus trichloride or phosphorus tribromide, the first compound, and the second compound flowing in the flow channel is set to 0.01 mL/min or more. The stirring efficiency can be improved and side reactions can be suppressed by appropriately controlling the reaction temperature and the flow rate.

Therefore, with respect to the organophosphorus compound according to the fifth aspect of the present disclosure, the desired organophosphorus compound can be produced at a low cost and with a high yield.

Also in the method for producing an organophosphorus compound according to the fifth aspect of the present disclosure, the relationship between the preferred flow rate and the inner diameter of the mixer of the flow reactor is the same as that of the method for producing an asymmetric phosphoric acid triester of the first embodiment described above.

That is, in a case where the inner diameter of the mixer of the flow reactor is defined as ID, the flow rate of each compound is preferably a value obtained by multiplying the above-mentioned preferred flow rate by (ID/0.25)².

### [Examples]

Hereinafter, the present disclosure will be described in more detail with reference to examples, but the present disclosure is not limited to the following examples.

### [Synthesis 1 of symmetric phosphoric acid triester]

### (Examples 1 to 8 and Comparative Example 1)

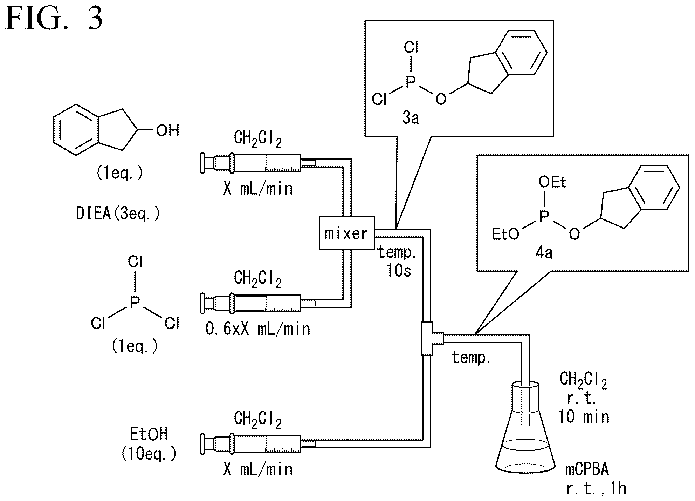

FIG. 3 shows a schematic diagram of the method for producing a symmetric phosphoric acid triester according to Examples 1 to 8.

### [Raw materials]

2-Hydroxyindane was used as R'OH. Ethanol was used as R²OH.

### «Examples 1 to 8»

A flow reactor composed of a PTFE tube (inner diameter: 0.80 mm, outer diameter: 1.59 mm) and a T-shaped or V-shaped mixer (inner diameter: 0.25 mm for both) shown in Table 1 was used as the flow reactor.

The solutions before the reaction were prepared in three portions.

A first solution was prepared by mixing 2-hydroxyindane, DIEA, and dichloromethane (DCM).

A second solution was prepared by mixing phosphorus trichloride and dichloromethane (DCM).

A third solution was prepared by mixing ethanol and dichloromethane (DCM).

The ratios of equivalents relative to 1.0 of 2-hydroxyindane were set to 3.0 for DIEA, 1.0 for phosphorus trichloride, and 10 for ethanol.

In order to synthesize a symmetric phosphoric acid triester, first, the first solution and the second solution were mixed in a T-shaped or V-shaped mixer shown in Table 1 and reacted at the reaction temperature shown in Table 1 for 10 seconds to obtain compound 3a. Subsequently and immediately, the reaction solution containing the compound 3a and the third solution were mixed using a new T-shaped mixer and reacted at the reaction temperature shown in Table 1 for 5 seconds to obtain compound 4a. In addition, the compound 4a was stored while continuously stirring the compound 4a at room temperature (25°C) for 10 minutes. Subsequently, the compound 4a and metachloroperbenzoic acid (mCPBA) were reacted at room temperature (25°C) for 1 hour to oxidize the compound 4a, thereby obtaining main product 5a. In addition, by-product 5b was also obtained by a side reaction.

The various solutions were pumped out using a syringe pump. The flow rate of each of the first solution and the third solution was as shown in Table 1, and the flow rate of the second solution was set to the flow rate shown in Table 1 × 0.6.

### <<Comparative Example 1»

In the same procedure as above, the first solution, the second solution, the third solution, and mCPBA were each added dropwise to a test tube stirred with a magnetic stirrer to obtain main product 5a and by-product 5b in a batch manner.

Each reaction for synthesizing a symmetric phosphoric acid triester by the production methods of Examples 1 to 8 and Comparative Example 1 is shown below.

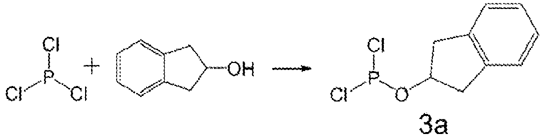

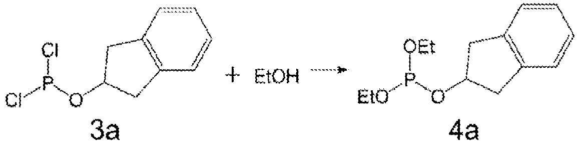

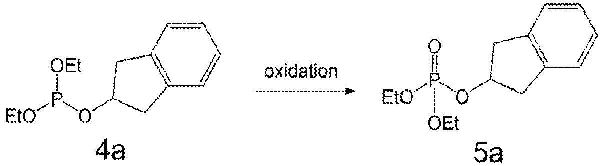

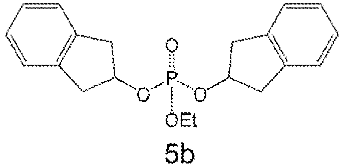

### [Analysis method]

The main product 5a and the by-product 5b were identified by NMR measurement. In addition, the yield measurements were carried out using HPLC-UV.

The NMR spectrum data are shown below, and the yields of the main product 5a and the by-product 5b are shown in Table 1.

### Main product 5a:

Colorless oil; IR (neat): 2980, 1653, 1541, 1457, 1260, 1165, 1026, 799, 744
¹H NMR (400 MHz, CDCl₃): δ = 7.24-7.17 (m, 4H), 5.25-5.22 (m, 1H), 4.13-4.06 (m, 4H), 3.29 (dd, J = 6.1, 16.6 Hz, 2H), 3.17 (dd, J = 3.6, 16.6 Hz, 2H), 1.33 (td, J = 0.6, 7.0 Hz, 6H)

### By-product 5b:

Colorless oil; IR (neat): 2957, 1653, 1541, 1458, 1260, 1008, 740, 669
¹H NMR (400 MHz, CDCl₃): δ = 7.23-7.16 (m, 8H), 5.24-5.20 (m, 2H), 4.11-4.04 (m, 2H), 3.26 (dd, J = 6.1, 16.7 Hz, 4H), 3.15 (dd, J = 3.6, 16.7 Hz, 4H), 1.31 (t, J = 7.1 Hz, 3H)

**[Table 1]**

| | Reaction temperature [°C] | Flow rate [ml/min] | Mixer | Yield | |
|---|---|---|---|---|---|
| | | | | 5a (desired) | 5b (undesired) |
| Example 1 | -10 | 2.0 | T-shaped | 68 | 4 |
| Example 2 | 0 | 2.0 | T-shaped | 72 | 5 |
| Example 3 | 10 | 2.0 | T-shaped | 75 | 5 |
| Example 4 | 20 | 2.0 | T-shaped | 76 | 5 |
| Example 5 | 30 | 2.0 | T-shaped | 76 | 5 |
| Example 6 | 20 | 1.0 | T-shaped | 60 | 10 |
| Example 7 | 20 | 2.0 | V-shaped | 79 | 5 |
| Example 8 | 20 | 4.0 | V-shaped | 79 | 5 |
| Comparative Example 1 | 20 | Batch | | 50 | 21 |

As shown in Table 1, in a case where the symmetric phosphoric acid triester was produced by the production method of Examples, the yield of the main product 5a was higher than that of the production method of Comparative Example.

### [Synthesis 2 of symmetric phosphoric acid triester]

### (Examples 9 to 14)

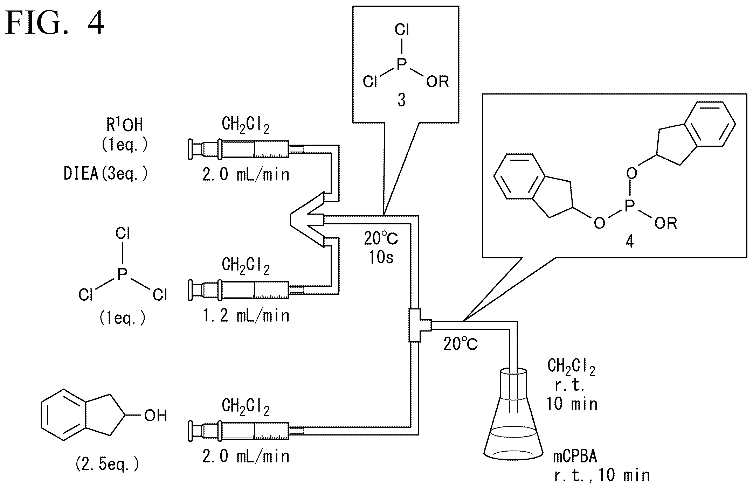

FIG. 4 shows a schematic diagram of the method for producing a symmetric phosphoric acid triester according to Examples 9 to 14.

### [Raw materials]

1-Butanol (Example 9), 2-butanol (Example 10), tert-butanol (Example 11), allyl alcohol (Example 12), 2-[[tert-butyl(dimethyl)silyl]oxy]ethanol (Example 13), and tert-butyl hydroxyacetate (Example 14) were used as R'OH.

### 2-Hydroxyindane was used as R²OH (Examples 9 to 14).

A flow reactor composed of a PTFE tube (inner diameter: 0.80 mm, outer diameter: 1.59 mm) and a T-shaped mixer and a V-shaped mixer (inner diameter: 0.25 mm for both) was used as the flow reactor.

### The solutions before the reaction were prepared in three portions.

A first solution was prepared by mixing any one of R¹OH's described above, DIEA, and DCM.

A second solution was prepared by mixing phosphorus trichloride and DCM.

A third solution was prepared by mixing 2-hydroxyindane and DCM.

The ratios of equivalents relative to 1.0 of R¹OH were set to 3.0 for DIEA, 1.0 for phosphorus trichloride, and 2.5 for 2-hydroxyindane.

In order to synthesize a symmetric phosphoric acid triester, first, the first solution and the second solution were mixed in a V-shaped mixer and reacted at 20°C for 10 seconds to obtain a compound 3. Subsequently and immediately, the reaction solution containing the compound 3 and the third solution were mixed using a new T-shaped mixer and reacted at 20°C for 5 seconds to obtain compound 4. In addition, the compound 4 was stored while continuously stirring the compound 4 at room temperature (25°C) for 10 minutes. Subsequently, the compound 4 and mCPBA were reacted at room temperature (25°C) for 10 minutes to oxidize the compound 4, thereby obtaining each of main products 5c to 5h.

The various solutions were pumped out using a syringe pump. The flow rate of each of the first solution and the third solution was set to 2.0 mL/min, and the flow rate of the second solution was set to 1.2 mL/min.

The main products 5c to 5h were identified by NMR measurement. In addition, the yield measurements were carried out using HPLC-UV.

The NMR spectrum data, and the structures and yields of the main products 5c to 5h are shown below.

The relationship between the method for producing a symmetric phosphoric acid triester according to Examples 9 to 14 and the main product to be obtained is as follows.

Example 9: main product 5c, Example 10: main product 5d, Example 11: main product 5e, Example 12: main product 5f, Example 13: main product 5g, Example 14: main product 5h.

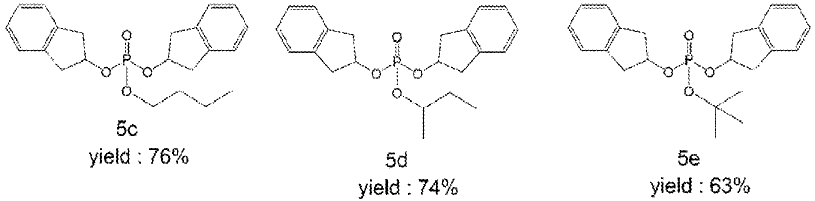

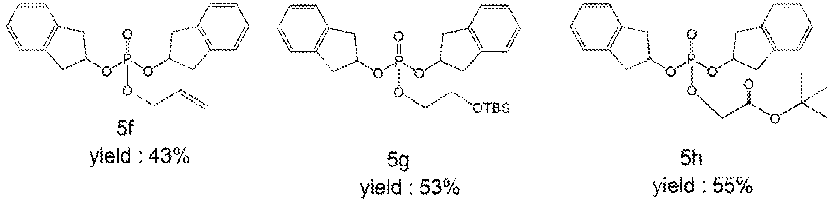

5c: n-Butyl bis(indan-2-yl)phosphate
Purification method: column chromatography (Hexane: Ethyl acetate = 3:2)
Colorless oil; IR (neat): 2956, 1460, 1273, 1004, 740
¹H NMR (400 MHz, CDCl₃): δ = 7.27-7.17 (m, 8H), 5.24-5.20 (m, 2H), 4,03-3.98 (m, 2H), 3.29-3.23 (m, 4H), 3.15 (dd, J = 3.2, 16.9 Hz, 4H), 1.66-1.62 (m, 2H), 1.42-1.36 (m, 2H), 0.91 (t, J = 7.3 Hz, 3H)

5d: s-Butyl bis(indan-2-yl)phosphate
Purification method: column chromatography (Hexane: Ethyl acetate = 3: 2)
Colorless oil; IR (neat): 2968, 1460, 1261, 994, 740
¹H NMR (400 MHz, CDCl₃): δ = 7.18-7.09 (m, 8H), 5.15-5.13 (m, 2H), 4.39-4.33 (m, 1H), 3.21-3.15 (m, 4H), 3.10-3.06 (m, 4H), 1.63-1.49 (m, 2H), 1.22 (d, J = 6.4 Hz, 3H), 0.85 (t, J = 7.2 Hz, 3H)

5e: t-Butyl bis(indan-2-yl)phosphate
Purification method: column chromatography (Hexane: Ethyl acetate = 3: 2)
Colorless oil; IR (neat): 2977, 1460, 1276, 994, 739
¹H NMR (400 MHz, CDCl₃): δ = 7.26-7.16 (m, 8H), 5.20-5.17 (m, 2H), 3.28-3.13 (m, 8H), 1.48 (s, 9H)

5f: Bis(indan-2-yl) 2-propen-1-ylphosphate
Purification method: column chromatography (Hexane:Ethylacetate = 3:2) and Gel Permeation Chromatography
Colorless oil; IR (neat): 2954, 1459, 1277, 988, 930, 739
¹H NMR (400 MHz, CDCl₃): δ = 7.23-7.15 (m, 8H), 5.97-5.87 (m, 1H), 5.37-5.32 (m, 1H), 5.27-5.20 (m, 3H), 4.52-4.48 (m, 2H), 3.28-3.21 (m, 4H), 3.15 (dd, J = 4.1, 16.5 Hz)

5g: 3-[[tert-Butyl(dimethyl)silyl]oxy]propan-1-yl bis(indan-2-yl)phosphate
Purification method: column chromatography (Hexane:Diethylether = 1:4)
Colorless oil; IR (neat): 2954, 1467, 1259, 1011, 837, 741
¹H NMR (400 MHz, CDCl₃): δ = 7.23-7.16 (m, 8H), 5.25-5.19 (m, 2H), 4.11 (q, J = 6, 5 Hz), 3.69 (t, J = 6.0 Hz), 3.26 (dd, J = 6.1, 16.7 Hz), 3.15 (dd, J = 3.6, 16.7 Hz), 1.85 (q, J = 6.1 Hz), 0.88 (s, 9H), 0.03 (s, 6H)

5h: tert-Butyl glycolatylbis(indan-2-yl)phosphate
Purification method: column chromatography (Hexane:Diethylether = 1:4)
Colorless oil; IR (neat): 2927, 1755, 1460, 1367, 1278, 1163, 1106, 1005, 741
¹H NMR (400 MHz, CDCl₃): δ = 7.23-7.16 (m, 8H), 5.33-5.27 (m, 2H), 4.43 (d, J = 10.3 Hz, 2H), 3.27 (dd, J = 6.0, 16.8 Hz, 2H), 3.20-3.15 (m, 4H), 1.48 (s, 9H)

As described above, in a case where the symmetric phosphoric acid triester was produced by the production methods of Examples, the main products 5c to 5h could be obtained with a high yield.

### [Synthesis 1 of asymmetric phosphoric acid triester]

### (Examples 15 to 25 and Comparative Example 2)

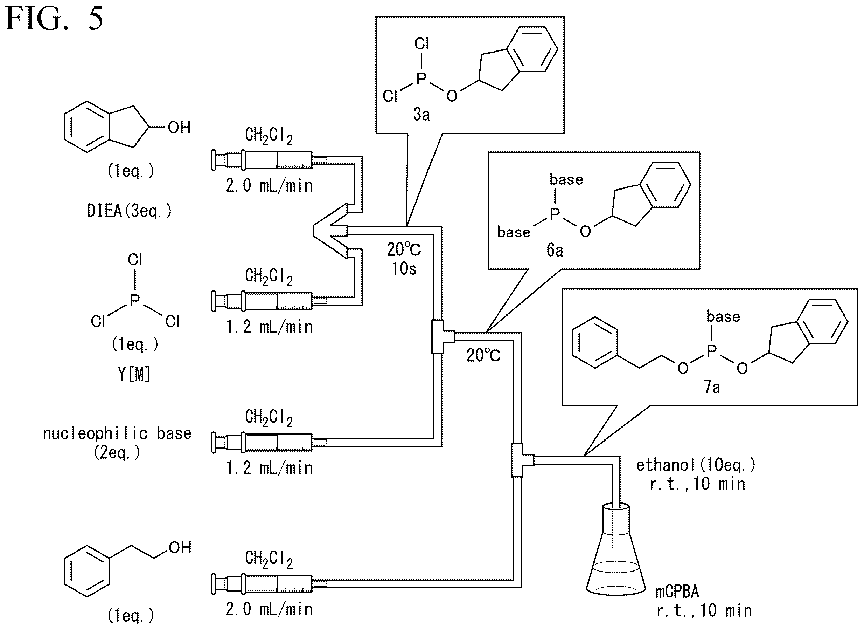

FIG. 5 shows a schematic diagram of the method for producing an asymmetric phosphoric acid triester according to Examples 15 to 25. FIG. 5 shows, as an example, the production method using N,N-diisopropylethylamine (DIEA) as the base. In addition, "base" in the compound represents a group obtained by removing one hydrogen atom from a nucleophilic base.

### [Raw materials]

In Examples 15 to 25, 2-hydroxyindane was used as R'OH, 2-phenylethanol (phenethyl alcohol) was used as R²OH, and ethanol was used as R³OH.

N,N-diisopropylethylamine (i-Pr₂NEt), 2,6-lutidine, N-methylmorpholine (NMM), N-ethylmorpholine, N-benzyldimethylamine (Me₂NBn), or tributylamine (n-Bu₃N) was used as the base (non-nucleophilic base).

Pyridine, imidazole, N-methylimidazole (NMI), 4-methylimidazole, or 4-dimethylaminopyridine (DMAP) was used as the nucleophilic base.

### «Examples 15 to 25»

A flow reactor composed of a PTFE tube (inner diameter: 0.80 mm, outer diameter: 1.59 mm) and a T-shaped mixer and a V-shaped mixer (inner diameter: 0.25 mm for both) was used as the flow reactor.

The solutions before the reaction were prepared in four portions.

A first solution was prepared by mixing 2-hydroxyindane, the base shown in Table 2, and dichloromethane.

A second solution was prepared by mixing phosphorus trichloride and DCM.

A third solution was prepared by mixing the nucleophilic base shown in Table 2 and DCM.

A fourth solution was prepared by mixing 2-phenylethanol and DCM.

The ratios of equivalents relative to 1.0 of 2-hydroxyindane were set to 3.0 for the base, 1.0 for phosphorus trichloride (0.5 in Examples 23 to 28), 2.0 for the nucleophilic base, 1.0 for 2-phenylethanol, and 10 for ethanol.

The concentration Y [M] in Table 2 is the molar concentration of phosphorus trichloride.

In order to synthesize an asymmetric phosphoric acid triester, first, the first solution and the second solution were mixed in a V-shaped mixer and reacted at 20°C for 10 seconds to obtain compound 3a. Subsequently, immediately, the reaction solution containing the compound 3a and the third solution were mixed using a new T-shaped mixer and reacted at 20°C for 10 seconds to obtain compound 6a. Subsequently, immediately, the reaction solution containing the compound 6a and the fourth solution were mixed using a new T-shaped mixer and reacted at 20°C for 5 seconds to obtain compound 7a. Subsequently, the reaction solution containing the compound 7a was allowed to flow into a beaker containing ethanol, followed by reaction at room temperature (25°C) for 10 minutes to obtain compound 7ab. Subsequently, the compound 7ab and mCPBA were reacted at room temperature (25°C) for 10 minutes to oxidize the compound 7ab, thereby obtaining main product 8a. In addition, by-products 8b and 8c were obtained by a side reaction.

The various solutions were pumped out using a syringe pump. The flow rate of each of the first solution and the fourth solution was set to 2.0 mL/min, and the flow rate of each of the second solution and the third solution was set to 1.2 mL/min.

### <<Comparative Example 2>>

In the same procedure as above, the first solution, the second solution, the third solution, the fourth solution, ethanol, and mCPBA were each added dropwise to a test tube stirred with a magnetic stirrer to obtain a main product 8a and by-products 8b and 8c in a batch manner.

Each reaction for synthesizing a symmetric phosphoric acid triester by the production methods of Examples 15 to 25 and Comparative Example 2 is shown below.

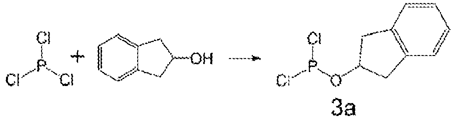

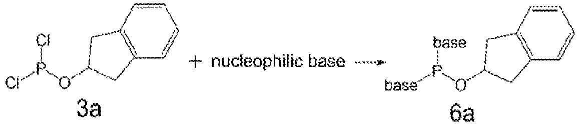

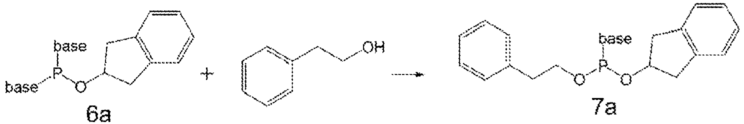

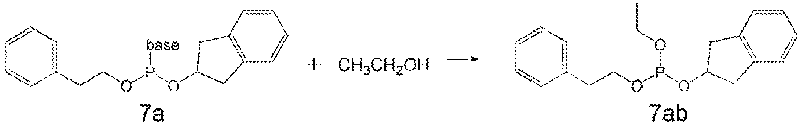

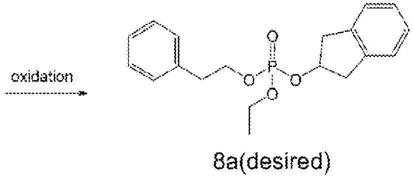

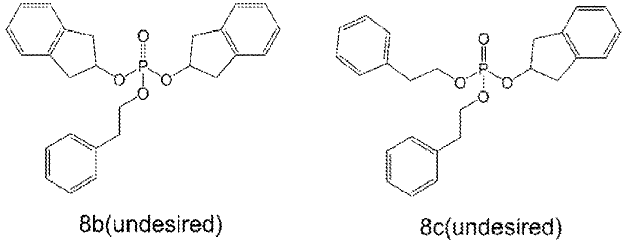

### [Analysis method]

The main product 8a, and the by-products 8b and 8c were identified by NMR measurement. In addition, the yield measurements were carried out using HPLC-UV.

The NMR spectrum data is shown below, and the yields of the main product 8a and the by-products 8b and 8c are shown in Tables 2 and 3.

### Main product 8a:

Purification method: preparative TLC (Toluene:EtOAc = 4:1)
¹H NMR (400 MHz, CDCl₃): δ = 7.31-7.16 (m, 9H), 5.16-5.11 (m, 1H), 4.22 (q, J = 7.0 Hz, 2H), 4.01 (t, 7.3 Hz, 2H), 3.25-3.06 (m, 4H), 2.98 (t, J = 7.0 Hz, 2H), 1.27 (t, J = 7.1 Hz, 3H)
³¹P NMR (162 MHz, CDCl₃): δ = -1.17

### By-product 8b:

Purification method: preparative TLC (Toluene:EtOAc = 4:1)
¹H NMR (400 MHz, CDCl₃): δ = 7.31-7.15 (m, 13H), 5.15-5.11 (m, 2H), 4.20 (q, J = 6.9 Hz, 2H), 3.22-3.04 (m, 8H), 2.98-2.91 (m, 2H)
³¹P NMR (162 MHz, CDCl₃): δ = -1.88

### By-product 8c:

Purification method: preparative TLC (Toluene:EtOAc = 4:1)
¹H NMR (400 MHz, CDCl₃): δ = 7.30-7.15 (m, 14H), 5.08-5.03 (m, 1H), 4.17-4.10 (m, 4H), 3.18-3.01 (m, 4H), 2.92 (t, J = 7.1 Hz, 4H)
³¹P NMR (162 MHz, CDCl₃): δ = -1.37

**[Table 2]**

| | Base | Concentration Y [M] | Nucleophilic base | Yield | | |
|---|---|---|---|---|---|---|
| | | | | 8a (desired) | 8b (undesired) | 8c (undesired) |
| Example 15 | i-Pr₂NEt | 0.2 | pyridine | 30 | 14 | 4 |
| Example 16 | i-Pr₂NEt | 0.2 | imidazole | 56 | 8 | 2 |
| Example 17 | i-Pr₂NEt | 0.2 | NMI | 32 | 17 | 7 |
| Example 18 | i-Pr₂NEt | 0.2 | 4-methylimidazole | 53 | 10 | 2 |
| Example 19 | i-Pr₂NEt | 0.2 | DMAP | 28 | 21 | 3 |
| Comparative Example 2 | i-Pr₂NEt | 0.2 | imidazole | 5 | 22 | 36 |

**[Table 3]**

| | Base | Concentration Y [M] | Nucleophilic base | Yield | | |
|---|---|---|---|---|---|---|
| | | | | 8a (desired) | 8b (undesired) | 8c (undesired) |
| Example 20 | i-Pr₂NEt | 0.1 | imidazole | 41 | 14 | 4 |
| Example 21 | lutidine | 0.1 | imidazole | 36 | 14 | 5 |
| Example 22 | NMM | 0.1 | imidazole | 45 | 12 | 1 |
| Example 23 | N-ethylmorph oline | 0.1 | imidazole | 42 | 13 | 2 |
| Example 24 | Me₂NBn | 0.1 | imidazole | 43 | 12 | 3 |
| Example 25 | n-Bu₃N | 0.1 | imidazole | 40 | 12 | 7 |

As shown in Tables 2 and 3, in a case where the asymmetric phosphoric acid triester was produced by the production method of Examples, the yield of the main product 8a was higher than that of the production method of Comparative Example.

### [Synthesis 2 of asymmetric phosphoric acid triester]

### (Examples 26 to 33)

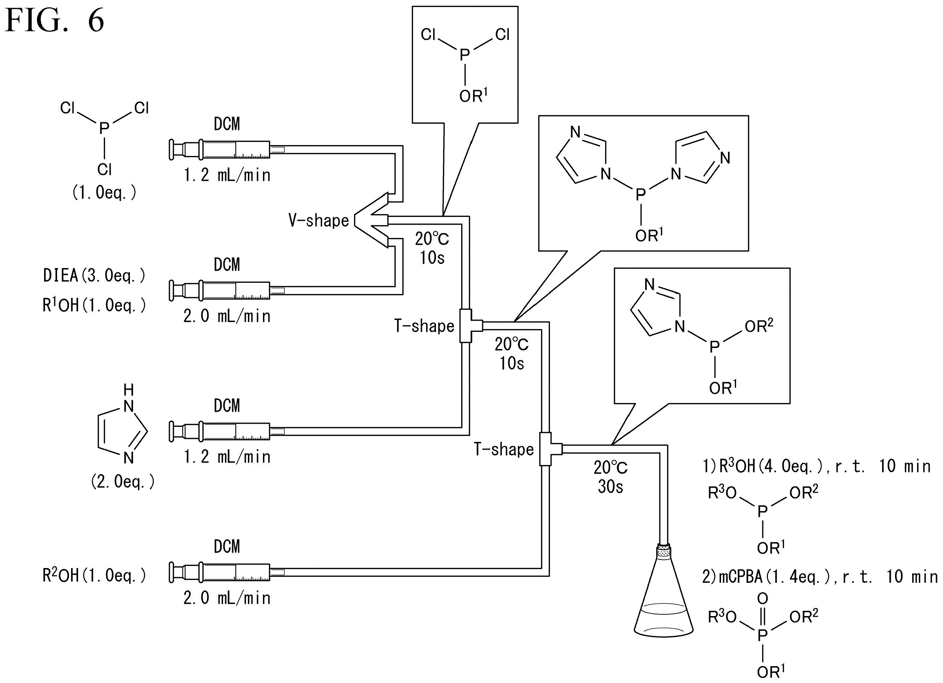

FIG. 6 shows a schematic diagram of the method for producing an asymmetric phosphoric acid triester according to Examples 26 to 33.

### [Raw materials]

In Example 26, 2-hydroxyindane was used as R¹OH, ethanol was used as R²OH, and phenol was used as R³OH.

In Example 27, 1-butanol was used as R¹OH, 2-hydroxyindane was used as R²OH, and phenol was used as R³OH.

In Example 28, 2-butanol was used as R¹OH, 2-hydroxyindane was used as R²OH, and phenol was used as R³OH.

In Example 29, 2-hydroxyindane was used as R¹OH, allyl alcohol was used as R²OH, and 2-methoxy-ethan-1-ol was used as R³OH.

In Example 30, 2-hydroxyindane was used as R¹OH, propargyl alcohol was used as R²OH, and 2-methoxy-ethan-1-ol was used as R³OH.

In Example 31, 2-hydroxyindane was used as R¹OH, tert-butyl(2-hydroxyethyl)carbamate was used as R²OH, and ethanol was used as R³OH.

In Example 32, 2-hydroxyindane was used as R¹OH, methyl[[(9H-fluorenyl)methoxy] carbonyl] serinate was used as R²OH, and ethanol was used as R³OH.

In Example 33, 3-benzoyl-1-[4-hydroxy-5-(tert-butyldimethylsilyloxymethyl)tetrahydrofuran-2-yl)-5-methylpyrimidine-2,4(1H,3H)-dione was used as R¹OH, 5-(3-benzoyl-5-methyl-2,4-dioxo-3,4-dihydropyrimidin-1(2H)-yl)-2-(hydroxymethyl)tetrahydrofuran-3-ylbenzoate was used as R²OH, and 2-cyanoethanol was used as R³OH.

A flow reactor composed of a PTFE tube (inner diameter: 0.80 mm, outer diameter: 1.59 mm) and V-shaped and T-shaped mixers (inner diameter: 0.25 mm for both) was used as the flow reactor.

### The solutions before the reaction were prepared in four portions.

A first solution was prepared by mixing any one of R¹OH's described above, DIEA, and DCM.

### A second solution was prepared by mixing phosphorus trichloride and DCM.

A third solution was prepared by mixing imidazole and DCM.

A fourth solution was prepared by mixing any one of R²OH's described above and DCM.

The ratios of equivalents relative to 1.0 of R¹OH were set to 3.0 for DIEA, 1.0 for phosphorus trichloride, 1.0 for R²OH, 2.0 for imidazole, 4.0 for R³OH, and 1.4 for mCPBA which will be described later.

In order to synthesize an asymmetric phosphoric acid triester, first, the first solution and the second solution were mixed in a V-shaped mixer and reacted at 20°C for 10 seconds to obtain a compound 1x. Subsequently and immediately, the reaction solution containing the compound 1x and the third solution were mixed using a new T-shaped mixer and reacted at 20°C for 10 seconds to obtain compound 2x. Subsequently and immediately, the reaction solution containing the compound 2x and the fourth solution were mixed using a new T-shaped mixer and reacted at 20°C for 30 seconds to obtain compound 3x. Subsequently, the reaction solution containing the compound 3x was allowed to flow into a beaker containing R³OH, followed by reaction at room temperature (25°C) for 10 minutes to obtain compound 4x. Subsequently, the compound 4x and mCPBA were reacted at room temperature (25°C) for 10 minutes to oxidize the compound 4x, thereby obtaining each of main products 1y to 8y.

The various solutions were pumped out using a syringe pump. The flow rate of each of the second solution and the fourth solution was set to 2.0 mL/min, and the flow rate of each of the first solution and the third solution was set to 1.2 mL/min.

Each reaction for synthesizing an asymmetric phosphoric acid triester by the production methods of Examples 26 to 33 is shown below.

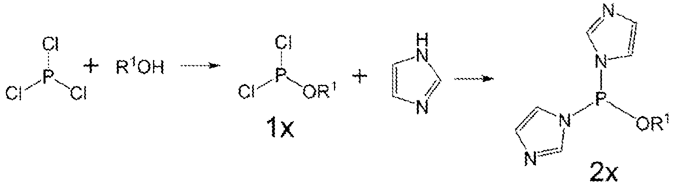

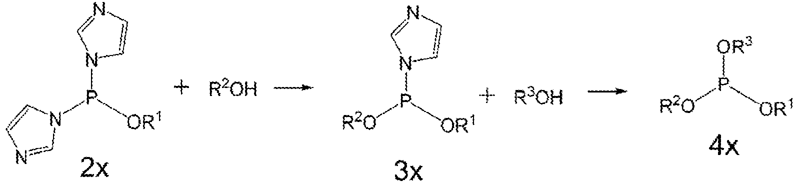

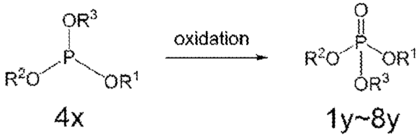

The main products 1y to 8y were identified by NMR measurement. In addition, the yield measurements were carried out using HPLC-UV.

The NMR spectrum data, and the structures and yields of the main products 1y to 8y are shown below.

The relationship between the method for producing an asymmetric phosphoric acid triester according to Examples 26 to 33 and the main product to be obtained is as follows.

Example 26: main product 1y, Example 27: main product 2y, Example 28: main product 3y, Example 29: main product 4y, Example 30: main product 5y, Example 31: main product 6y, Example 32: main product 7y, Example 33: main product 8y.

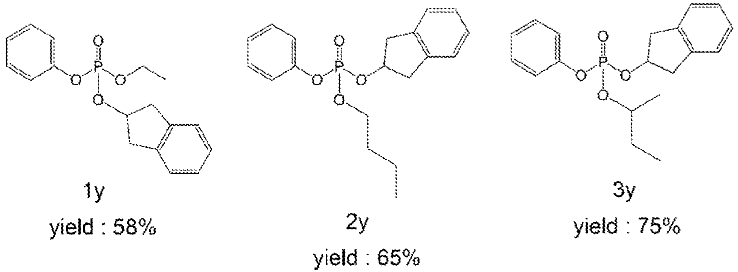

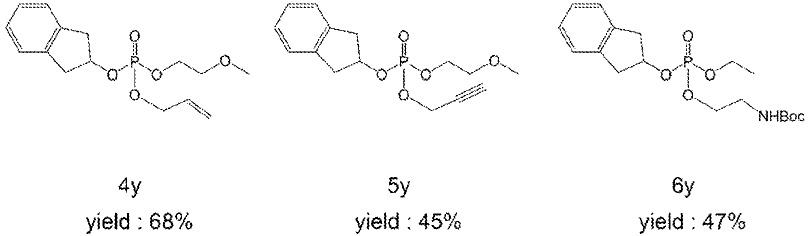

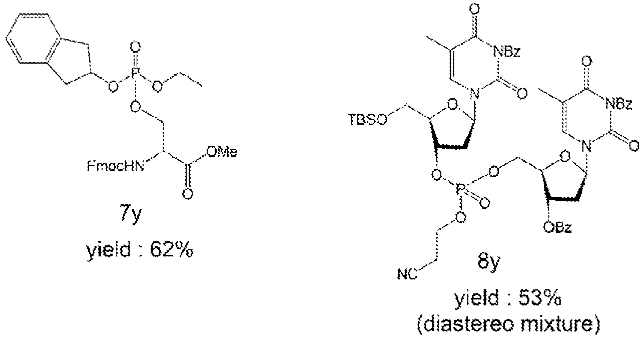

1y: Ethyl indan-2-yl phenyl phosphate
Purification method: column chromatography (Hexane:EtOAc = 2:1)
Colorless oil; IR (neat): 2981, 1592, 1490, 1279, 1210, 1164, 1009, 942, 742, 689
¹H NMR (400 MHz, CDCl₃): δ = 7.34-7.15 (m, 9H), 5.38-5.32 (m, 1H), 4.24-4.15 (m, 2H), 3.34-3.09 (m, 4H), 1.33 (dt, J = 1.1, 7.1 Hz, 3H)
¹³C NMR (125 MHz, CDCl₃): δ = 150.84, 150.78, 139.83, 139.80, 129.8, 127.0, 125.1, 124.77, 124.75, 120.21, 120.17, 80.15, 80.11, 64.81, 64.76, 40.91, 40.87, 40.79, 40.75, 16.2 (d, J = 6.8 Hz)
³¹P NMR (162 MHz, CDCl₃): δ = -6.97

2y: n-Butyl indan-2-yl phenyl phosphate
Purification method: column chromatography (Hexane:EtOAc = 2:1)
Colorless oil; IR (neat): 2960, 1592, 1490, 1281, 1211, 1009, 943, 742, 689
¹H NMR (400 MHz, CDCl₃): δ = 7.34-7.15 (m, 9H), 5.36-5.33 (m, 1H), 4.15-4.09 (m, 2H), 3.34-3.09 (m, 4H), 1.67-1.61 (m, 2H), 1.40-1.35 (m, 2H), 0.90 (t, J = 7.9 Hz, 3H)
¹³C NMR (100 MHz, CDCl₃): δ = 150.9 (d, J = 7.1 Hz), 139.8 (d, J = 2.9 Hz), 129.8, 127.0, 125.1, 124.8 (d, J = 1.8 Hz), 120.2 (d, J = 5.0 Hz), 80.1 (d, J = 6.0 Hz), 68.4 (d, J = 6.5 Hz), 40.9 (dd, J = 7.4, 18.1 Hz), 32.3 (d, J = 6.9 Hz), 18.7, 13.6
³¹P NMR (162 MHz, CDCl₃): δ = -6.82

3y: s-Butyl indan-2-yl phenyl phosphate
Purification method: column chromatography (Hexane:EtOAc = 2:1)
Colorless oil; IR (neat): 2973, 1592, 1490, 1278, 1211, 997, 940, 741, 689
¹H NMR (400 MHz, CDCl₃): δ = 7.33-7.13 (m, 9H), 5.35-5.32 (m, 1H), 4.59-4.50 (m, 1H), 3.29-3.09 (m, 4H), 1.70-1.58 (m, 2H), 1.34 (d, J = 6.2 Hz, 3H), 1.27 (d, J = 6.2 Hz, 3H), 0.93 (t, J = 7.5 Hz, 3H), 0.88 (t, J = 7.5 Hz, 3H)
¹³C NMR (125 MHz, CDCl₃): δ = 151.0 (dd, J = 3.3, 10.5 Hz), 139.9 (m), 129.7, 127.0, 125.0, 124.7 (d, J = 2.9 Hz), 120.2 (t, J = 4.8 Hz), 79.9 (dd, J = 2.5, 6.2 Hz), 78.6 (d, J = 6.6 Hz), 40.9 (d, J = 5.4 Hz), 40.7 (dd, J = 2.4, 8.1 Hz), 30.4 (dd, J = 6.2, 10.6 Hz), 21.1 (dd, J = 3.5, 16.5 Hz), 9.5 (d, J = 15.9 Hz), 0.1
³¹P NMR (162 MHz, CDCl₃): δ = -7.52, -7.63

4y: Indan-2-yl 2-methoxyethan-1-yl 2-propen-1-yl phosphate
Purification method: preparative TLC (Hexane:EtOAc = 2:3)
¹H NMR (400 MHz, CDCl₃): δ = 7.24-7.17 (m, 4H), 5.98-5.89 (m, 1H), 5.36 (ddd, J = 1.4, 3.0, 17.2 Hz, 1H), 5.30-5.23 (m, 2H), 4.53 (t, J = 6.4 Hz, 2H), 4.20-4.12 (m, 2H), 3.62-3.58 (t, J = 4.8 Hz, 2H), 3.38 (s, 3H), 3.31-3.14 (m, 4H)

5y: Indan-2-yl 2-methoxyethan-1-yl 2-propyn-1-yl phosphate
Purification method: column chromatography (Hexane:EtOAc = 2:3)
Colorless oil; IR (neat): 3217, 2924, 1457, 1275, 1033, 843, 747
¹H NMR (400 MHz, CDCl₃): δ = 7.24-7.17 (m, 4H), 5.33-5.26 (m, 1H), 4.67 (dd, J = 2.3, 10.1 Hz, 2H), 4.21-4.17 (m, 2H), 3.60 (t, J = 4.6 Hz, 2H), 3.38 (s, 3H), 3.30 (dd, J = 6.0, 16.9 Hz, 2H), 3.21 (dd, J = 3.4, 16.7 Hz, 2H), 2.55 (t, J = 2.3 Hz, 1H)

6y: 2-(N-(tert-Butoxycarbonyl)-amino)-ethan-1-yl ethyl indan-1-yl phosphate
Purification method: column chromatography (Hexane:EtOAc = 2:3)
Colorless oil; IR (neat): 3310, 2976, 1714, 1508, 1457, 1268, 1171, 1027, 741
¹H NMR (400 MHz, CDCl₃): δ = 7.25-7.18 (m, 4H), 5.27-5.23 (m, 1H), 5.02 (s, 1H), 4.15-4.04 (m, 4H), 3.39, (d, J = 5.0 Hz, 2H), 3.29 (dd, J = 16.5, 6.0 Hz, 2H), 3.17 (dd, J = 16.7, 3.4 Hz, 2H), 1.44 (s, 9H), 1.34 (t, J = 6.4 Hz, 3H)

7y: Ethyl 2-(N-(9-fluorenylmethyloxycarbonyl)-amino)-3-methoxycarbonylethan-1-yl indan-1-yl phosphate
Purification method: column chromatography (Hexane:EtOAc = 2:3)
Colorless oil; IR (neat): 3279, 2953, 1723, 1541, 1449, 1261, 1213, 1026, 741
¹H NMR (400 MHz, CDCl₃): δ = 7.77-7.73 (m, 2H), 7.62-7.57 (m, 2H), 7.41-7.16 (m, 8H), 5.96-5.93 (m, 1H), 5.27-5.21 (m, 1H), 4.60-4.58 (m, 1H), 4.49-4.40 (m, 2H), 4.36-4.28 (m, 2H), 4.26-4.18 (m, 1H), 4.13-4.02 (m, 2H), 3.82-3.69 (m, 3H), 3.31-3.23 (m, 2H), 3.21-3.13 (m, 2H), 1.36-1.26 (m, 3H)

8y: N3-Bz-5'-O-TBS-thymidine-3'-yl 2-cyanoethan-1-yl N3-Bz-3'-O-Bz-thymidine-5'-yl phosphate
Purification method: column chromatography (Hexane:EtOAc = 1:4)
Colorless oil; IR (neat): 2954, 1749, 1703, 1662, 1448, 1270, 1101, 1011, 835, 762
¹H NMR (400 MHz, CDCl₃): δ = 7.99 (d, J = 8.0 Hz, 2H), 7.95-7.92 (m, 4H), 7.66-7.57 (m, 3H), 7.52-7.42 (m, 8H), 6.42 (dd, J = 5.6, 8.0 Hz, 1H), 6.38 (dd, J = 4.0, 9.2 Hz, 1H), 5.53 (brd, J = 6.9 Hz, 1H), 5.11 (brt, J = 5.5 Hz, 1H), 4.52-4.40 (m, 2H), 4.37 (brs, 1H), 4.32 (brs, 1H), 4.31 (t, J = 6.0 Hz, 3H), 3.95 (brs, 2H), 2.79 (t, J = 6.0 Hz, 2H), 2.69-2.60 (m, 2H), 2.46-2.38 (m, 1H), 2.30-2.22 (m, 1H), 2.00 (s, 3H), 1.97 (s, 3H), 0.95 (s, 9H), 0.16 (s, 3H), 0.15 (s, 3H)

As described above, in a case where the asymmetric phosphoric acid triester was produced by the production methods of Examples, the main products 1y to 8y could be obtained with a high yield.

### [Synthesis 1 of organophosphorus compound]

### (Example 34)

### [Raw materials]

2-Hydroxyindane was used as R¹OH. 2-Butanol was used as R²OH. Saturated saline was used as R³OH.

A flow reactor composed of a PTFE tube (inner diameter: 0.80 mm, outer diameter: 1.59 mm) and a T-shaped mixer and a V-shaped mixer (inner diameter: 0.25 mm for both) was used as the flow reactor.

### The solutions before the reaction were prepared in four portions.

A first solution was prepared by mixing 2-hydroxyindane, DIEA, and DCM.

A second solution was prepared by mixing phosphorus trichloride and DCM.

A third solution was prepared by mixing imidazole and DCM.

A fourth solution was prepared by mixing 2-butanol and DCM.

The amounts of equivalents relative to 1.0 of 2-hydroxyindane were set to 3.0 for DIEA, 1.0 for phosphorus trichloride, 2.0 for imidazole, and 1.0 for 2-butanol.

In order to synthesize an organophosphorus compound, first, the first solution and the second solution were mixed in a V-shaped mixer and reacted at 20°C for 10 seconds to obtain a compound 1p. Subsequently and immediately, the reaction solution containing the compound 1p and the third solution were mixed using a new T-shaped mixer and reacted at 20°C for 10 seconds to obtain compound 2p. Subsequently and immediately, the reaction solution containing the compound 2p and the fourth solution were mixed using a new T-shaped mixer and reacted at 20°C for 30 seconds to obtain compound 3p. Subsequently, the reaction solution containing the compound 3p was allowed to flow into a beaker containing 1 mL of saturated saline, followed by reaction at room temperature (25°C) for 10 minutes to obtain an organophosphorus compound 4p.

The various solutions were pumped out using a syringe pump. The flow rate of each of the first solution and the fourth solution was set to 2.0 mL/min, and the flow rate of each of the second solution and the third solution was set to 1.2 mL/min.

Each reaction for synthesizing an organophosphorus compound by the production method of Example 34 is shown below.

In addition, the organophosphorus compound 4p was identified by NMR measurement.

The structure and yield of the organophosphorus compound 4p and the NMR spectrum data are also shown below.

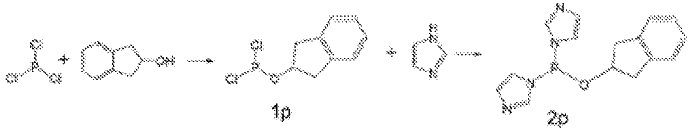

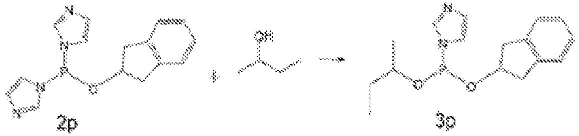

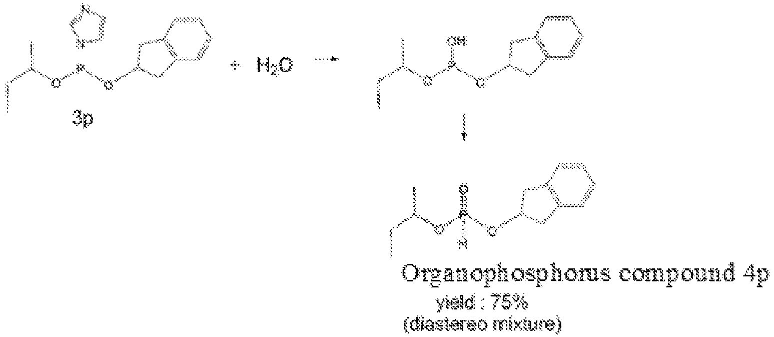

### Organophosphorus compound 4p:

Purification method: preparative TLC (Hexane:EtOAc = 3:2)
¹H NMR (400 MHz, CDCl₃): δ = 7.25-7.18 (m, 4H), 6.88 (d, J = 692 Hz, 1H), 5.35-5.30 (m, 1H), 4.54 (tq, J = 6.0, 6.4 Hz, 1H), 3.30 (dd, J = 6.2, 16.7 Hz, 2H), 3.17 (dd, J = 3.4, 16.7 Hz, 2H), 1.72-1.59 (m, 2H), 1.34 (dd, J = 6.4, 6.9 Hz, 3H), 0.95 (t, J = 7.3 Hz, 3H) (diastereomeric mixture)
³¹P NMR (162 MHz, CDCl₃): δ = 6.16, 6.51 (diastereomeric mixture)

As described above, in a case where the organophosphorus compound was produced by the production method of Example, the organophosphorus compound 4p could be obtained with a high yield.

### [Synthesis 3 of symmetric phosphoric acid triester]

### (Examples 35 to 40)

A symmetric phosphoric acid triester was produced in the same manner as in the section of [Synthesis 1 of symmetric phosphoric acid triester], except that R¹OH and R²OH were changed to the following raw materials.

In Example 35, n-butanol was used as R¹OH, and 2-phenylethanol (phenethyl alcohol) was used as R²OH.

In Example 36, 2-butanol was used as R¹OH, and 2-phenylethanol (phenethyl alcohol) was used as R²OH.

In Example 37, tert-butanol was used as R¹OH, and 2-phenylethanol (phenethyl alcohol) was used as R²OH.

In Example 38, 2-phenylethanol (phenethyl alcohol) was used as R¹OH, and n-butanol was used as R²OH.

In Example 39, 2-phenylethanol (phenethyl alcohol) was used as R¹OH, and 2-butanol was used as R²OH.

In Example 40, 2-phenylethanol (phenethyl alcohol) was used as R¹OH, and phenol was used as R²OH.

The main products 5i to 5n were identified by NMR measurement. In addition, the yield measurements were carried out using HPLC-UV

The NMR spectrum data, the precise mass measurement data, and the structures and yields of the main products 5i to 5n are shown below.

The relationship between the method for producing a symmetric phosphoric acid triester according to Examples 35 to 40 and the main product to be obtained is as follows.

Example 35: main product 5i, Example 36: main product 5j, Example 37: main product 5k, Example 38: main product 51, Example 39: main product 5m, Example 40: main product 5n.

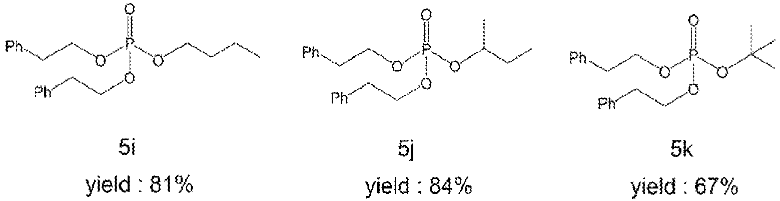

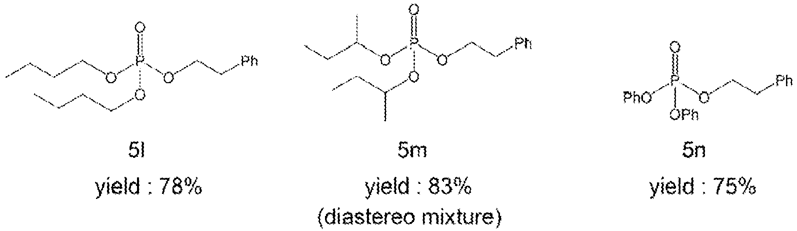

Main product 5i: Butyl diphenethyl phosphate
Purification method: preparative TLC (Toluene:EtOAc = 4:1)
Colorless oil; IR (neat): 2959, 1271, 1019, 746 cm⁻¹
¹H NMR (400 MHz, CDCl₃): δ = 7.31-7.26 (m, 4H), 7.23-7.18 (m, 6H), 4.19-4.11 (m, 4H), 3.87 (td, J = 6.8, 6.8 Hz, 2H), 2.94 (t, J = 7.2 Hz, 4H), 1.56 (tt, J = 6.8, 7.6 Hz, 2H), 1.53 (qt, J = 7.2, 7.6 Hz, 2H), 0.90 (t, J = 7.2 Hz, 3H)
¹³C NMR (125 MHz, CDCl₃): δ = 137.2, 129.0, 128.5, 126.6, 67.8 (d, J = 5.7 Hz), 67.5 (d, J = 6.6 Hz), 36.7 (d, J = 6.6 Hz), 32.2 (d, J = 6.7 Hz), 18.6, 13.5
³¹P NMR (162 MHz, CDCl₃): δ = -0.59
HRMS (ESI): calcd for [C₂₀H₂₇O₄P + Na]⁺ 385.1539, found 385.1539

Main product 5j: Diphenethyl 1-methylpropyl phosphate
Purification method: preparative TLC (Hexane:EtOAc = 3:1)
Colorless oil; IR (neat): 2969, 1269, 1006, 746 cm⁻¹
¹H NMR (400 MHz, CDCl₃): δ = 7.30-7.26 (m, 4H), 7.23-7.18 (m, 6H), 4.32 (dtq, J = 6.4, 6.4, 6.4 Hz, 1H), 4.17-4.11 (m, 4H), 2.94 (t, J = 7.2 Hz, 4H), 1.65-1.46 (m, 2H), 1.23 (d, J = 6.4 Hz, 3H), 0.88 (t, J = 7.4 Hz, 3H)
¹³C NMR (125 MHz, CDCl₃): δ = 137.2, 129.0, 128.4, 126.6, 77.4, 67.7 (d, J = 5.7 Hz), 36.7 (d, J = 7.6 Hz), 30.2 (d, J = 5.7 Hz), 20.9 (d, J = 3.8 Hz), 9.4
³¹P NMR (162 MHz, CDCl₃): δ = -1.34
HRMS (ESI): calcd for [C₂₀H₂₇O₄P + Na]⁺ 385.1539, found 385.1539

Main product 5k: 1,1-Dimethylethyl diphenethyl phosphate
Purification method: GPC
Colorless oil; IR (neat): 2975, 1271, 1006, 748
¹H NMR (400 MHz, CDCl₃): δ = 7.30-7.26 (m, 4H), 7.23-7.15 (m, 6H), 4.14-4.06 (m, 4H), 2.93 (t, J = 7.0 Hz, 4H), 1.41 (s, 9H)
¹³C NMR (125 MHz, CDCl₃): δ = 137.4, 129.0, 128.4, 126.6, 82.9 (d, J = 6.7 Hz), 67.5 (d, J = 5.8 Hz), 36.7 (d, J = 7.6 Hz), 29.7 (d, J = 4.8 Hz)
³¹P NMR (162 MHz, CDCl₃): δ = -5.09
HRMS (ESI): calcd for [C₂₀H₂₇O₄P + Na]⁺ 385.1539, found 385.1539

Main product 51: Dibutyl phenethyl phosphate
Purification method: preparative TLC (Toluene:EtOAc = 4:1) and GPC
Colorless oil; IR (neat): 2959, 1272, 1022, 745 cm⁻¹
¹H NMR (400 MHz, CDCl₃): δ = 7.32-7.27 (m, 2H), 7.24-7.18 (m, 3H), 4.23 (dt, J = 7.1, 7.1 Hz, 2H), 3.96 (dt, J = 6.7, 6.7 Hz, 2H), 3.95 (dt, J = 6.9, 6.9 Hz, 2H), 3.00 (t, J = 6.8 Hz, 2H), 1.61 (tt, J = 6.8, 7.2 Hz, 4H), 1.37 (tq, J = 7.2, 7.4 Hz, 4H), 0.92 (t, J = 7.4 Hz, 6H)
¹³C NMR (125 MHz, CDCl₃): δ = 137.2, 129.0, 128.5, 126.6, 67.8 (d, J = 5.7 Hz), 67.4 (d, J = 5.7 Hz), 36.8 (d, J = 7.7 Hz), 32.2 (d, J = 6.7 Hz), 18.6, 13.5
³¹P NMR (162 MHz, CDCl₃): δ = -0.29
HRMS (ESI): calcd for [C₁₆H₂₇O₄P + Na]⁺ 337.1539, found 337.1540

Main product 5m (diastereo mixture): Di-1-methylpropyl phenethyl phosphate
Purification method: preparative TLC (Toluene:EtOAc = 4:1)
Colorless oil; IR (neat): 2971, 1266, 1002, 745 cm⁻¹
¹H NMR (400 MHz, CDCl₃): δ = 7.31-7.26 (m, 2H), 7.24-7.18 (m, 3H), 4.37 (dtq, J = 5.8, 5.9, 6.4 Hz, 1H), 4.36 (dtq, J = 5.8, 5.9, 6.4 Hz, 1H), 4.24-4.17 (m, 2H), 2.99 (t, J = 7.2 Hz, 2H), 1.70-1.49 (m, 4H), 1.28 (d, J = 6.4 Hz, 3H), 1.26 (d, J = 6.4 Hz, 3H), 0.92 (t, J = 7.8 Hz, 3H), 0.90 (t, J = 7.8 Hz, 3H)
¹³C NMR (125 MHz, CDCl₃): δ = 137.7, 128.9, 128.3, 126.4, 76.2 (d, J = 5.7 Hz), 67.1 (d, J = 6.6 Hz), 35.9 (d, J = 7.6 Hz), 29.6 (d, J = 7.6 Hz), 20.7, 9.2
³¹P NMR (162 MHz, CDCl₃): δ = -1.71
HRMS (ESI): calcd for [C₁₆H₂₇O₄P + Na]⁺ 337.1539, found 337.1540

Main product 5n: Diphenyl phenethyl phosphate
Purification method: preparative TLC (Hexane:EtOAc = 4:1)
¹H NMR (400 MHz, CDCl₃): δ = 7.36-7.22 (m, 7H), 7.19-7.08 (m, 8H), 4.44 (td, J = 6.8, 7.6 Hz, 2H), 3.01 (t, J = 6.8 Hz, 2H)
¹³C NMR (125 MHz, CDCl₃): δ = 150.5 (d, J = 7.6 Hz), 136.6, 129.7, 129.0, 128.6, 126.8, 125.3, 120.0 (d, J = 4.8 Hz), 69.4 (d, J = 6.7 Hz), 36.6 (d, J = 7.6 Hz)
³¹P NMR (162 MHz, CDCl₃): δ = -11.41
HRMS (ESI): calcd for [C₂₀H₁₉O₄P + Na]⁺ 377.0913, found 377.0914

### [Synthesis 3 of asymmetric phosphoric acid triester]

### (Examples 41 to 45)

An asymmetric phosphoric acid triester was produced in the same manner as in the section of [Synthesis 2 of asymmetric phosphoric acid triester], except that R'OH, R²OH, and R³OH were changed to the raw materials described below.

### (Example 46)

An asymmetric phosphoric acid triester (asymmetric thiophosphoric acid triester) was produced in the same manner as in the section of [Synthesis 2 of asymmetric phosphoric acid triester], except that R¹OH, R²OH and R³OH were changed to the raw materials described below, and the reagent used for the oxidation was changed from mCPBA to xanthane hydride.

In Example 41, 2-hydroxyindane was used as R¹OH, 2-phenylethanol (phenethyl alcohol) was used as R²OH, and ethanol was used as R³OH.

In Example 42, 2-hydroxyindane was used as R¹OH, menthol was used as R²OH, and ethanol was used as R³OH.

In Example 43, 2-hydroxyindane was used as R¹OH, 2,2-dimethyl-1,3-dioxolane-4-methanol was used as R²OH, and phenol was used as R³OH.

In Example 44, 2-hydroxyindane was used as R¹OH, 2-methyl 2,3,4-tri-O-benzyl-α-D-glucopyranoside was used as R²OH, and phenol was used as R³OH.

In Example 45, 2-hydroxyindane was used as R¹OH, 1-O,2-O-dipalmitoyl-D-glycerol was used as R²OH, and ethanol was used as R³OH.

In Example 46, 2-hydroxyindane was used as R¹OH, 2-butanol was used as R²OH, and phenol was used as R³OH.

The main products 9y to 14y were identified by NMR measurement. In addition, the yield measurements were carried out using HPLC-UV.

The NMR spectrum data, the precise mass measurement data, and the structures and yields of the main products 9y to 14y are shown below.

The relationship between the method for producing an asymmetric phosphoric acid triester according to Examples 41 to 46 and the main product to be obtained is as follows.

Example 41: main product 9y, Example 42: main product 10y, Example 43: main product 11y, Example 44: main product 12y, Example 45: main product 13y, Example 46: main product 14y.

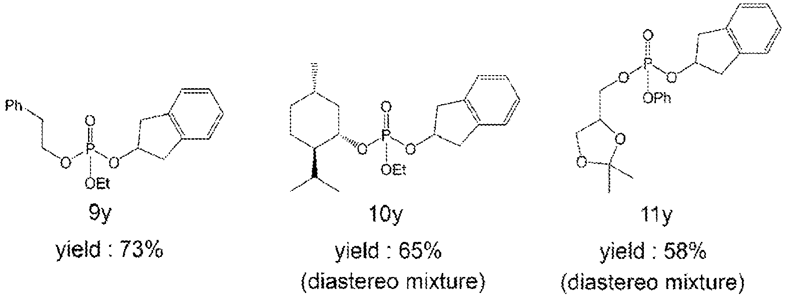

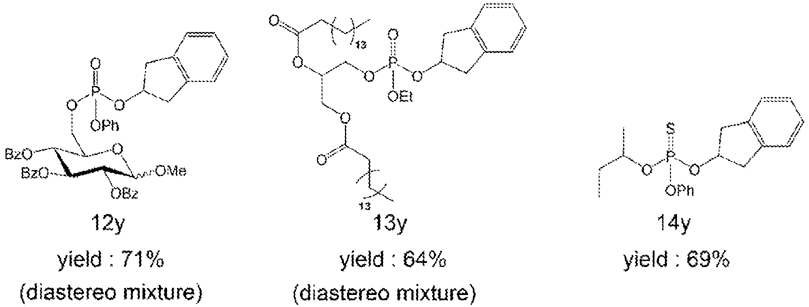

Main product 9y: Ethyl 2-indanyl phenethyl phosphate
Purification method: preparative TLC (Toluene:EtOAc = 4:1)
Colorless oil; IR (neat): 2959, 1276, 1015, 745 cm⁻¹
¹H NMR (400 MHz, CDCl₃): δ = 7.31-7.27 (m, 2H), 7.24-7.16 (m, 7H), 5.14 (ttd, J = 3.6, 6.0, 6.0 Hz, 1H), 4.22 (td, J = 7.2, 7.2 Hz, 2H), 4.05-3.97 (m, 2H), 3.25-3.16 (m, 2H), 3.14-3.06 (m, 2H), 2.98 (t, J = 7.2 Hz, 2H), 1.27 (dt, J = 0.8 Hz, 7.2 Hz, 3H)
¹³C NMR (125 MHz, CDCl₃): δ = 139.8, 137.2, 129.0, 128.5, 126.8, 126.6, 124.6, 79.0 (d, J = 5.7 Hz), 67.9 (d, J = 5.8 Hz), 63.7 (d, J = 5.7 Hz), 40.7 (d, J = 4.8 Hz), 40.6 (d, J = 4.7 Hz), 36.7 (d, J = 7.6 Hz), 16.1 (d, J = 6.7 Hz)
³¹P NMR (162 MHz, CDCl₃): δ = -1.125
HRMS (ESI): calcd for [C₁₉H₂₃O₄P + Na]⁺ 369.1226, found 369.1228

Main product 10y (diastereo mixture): Ethyl 2-indanyl (-)-p-menthane-3-yl phosphate
Purification method: preparative TLC (Hexane:EtOAc = 4:1)
Colorless oil; IR (neat): 2956, 1266, 1007, 743 cm⁻¹
¹H NMR (400 MHz, CDCl₃): δ = 7.24-7.22 (m, 2H), 7.19-7.16 (m, 2H), 5.26-5.18 (m, 1H), 4.20-4.12 (m, 1H), 4.11-4.03 (m, 2H), 3.28 (dd, J = 6.4, 16.4 Hz, 2H), 3.20-3.14 (m, 2H), 2.21 (brd, J = 12.4 Hz, 1H), 2.16-2.08 (m, 1H), 1.66 (brs, 1H), 1.64 (brs, 1H), 1.47-1.38 (m, 1H), 1.37-1.29 (m, 1H), 1.32 (t, J=7.2 Hz, 3H), 1.12 (ddd, J = 2.8, 11.2, 12.0 Hz, 1H), 1.05-0.95 (m, 1H), 0.90 (d, J = 6.4 Hz, 6H), 0.89 (d, J = 7.2 Hz, 6H), 0.86-0.82 (m, 1H), 0.79 (d, J = 6.8 Hz, 3H), 0.75 (d, J = 7.2 Hz, 3H)
¹³C NMR (125 MHz, CDCl₃): δ = 140.0, 126.8, 124.6, 79.3 (d, J = 6.7 Hz), 79.2 (d, J = 6.7 Hz), 78.8 (d, J = 5.7 Hz), 78.7 (d, J = 7.7 Hz), 63.5 (d, J = 4.8 Hz), 63.4 (d, J = 5.7 Hz), 48.4 (d, J = 6.7 Hz), 42.5, 40.8 (d, J = 5.7 Hz), 40.7 (d, J = 4.8 Hz), 34.0, 31.5, 25.5 (d, J = 4.8 Hz), 22.8 (d, J = 3.8 Hz), 21.9, 20.9, 16.2 (d, J = 5.7 Hz), 16.1 (d, J = 6.7 Hz), 15.6 (d, J = 3.8 Hz)
³¹P NMR (162 MHz, CDCl₃): δ = -1.55, -1.63
HRMS (ESI): calcd for [C₂₁H₃₃O₄P + Na]⁺ 403.2009, found 403.2008

Main product 11y (diastereo mixture): 2,2-Dimethyl-1,3-dioxolane-4-methyl 2-indanyl phenyl phosphate
Purification method: preparative TLC (Hexane:EtOAc = 3:2)
Colorless oil; IR (neat): 2925, 1281, 1212, 1015, 947, 749 cm⁻¹
¹H NMR (400 MHz, CDCl₃): δ = 7.35-7.31 (m, 2H), 7.25-7.16 (m, 7H), 5.37 (ddt, J = 3.0, 3.2, 6.0 Hz, 1H), 5.36 (ddt, J = 3.0, 3.2, 6.0 Hz, 1H), 4.29 (dq, J = 2.8, 6.0 Hz, 1H), 4.18-4.05 (m, 2H), 4.02 (dd, J = 6.4, 8.8 Hz, 2H), 3.79 (ddd, J = 2.8, 6.0, 8.8 Hz, 2H), 3.34-3.10 (m, 4H), 1.40 (s, 3H), 1.39 (s, 3H), 1.34 (s, 3H)
¹³C NMR (125 MHz, CDCl₃): δ = 150.5 (d, J = 6.7 Hz), 139.6, 129.7, 126.9, 125.2, 124.6, 120.0 (d, J = 4.7 Hz), 109.9, 80.4 (d, J = 5.7 Hz), 73.9, 73.8, 68.0 (d, J = 6.7 Hz), 67.9 (d, J = 5.7 Hz), 40.7 (d, J = 4.8 Hz), 40.6 (d, J = 5.7 Hz), 26.7, 25.2.
³¹P NMR (162 MHz, CDCl₃): δ = -6.39, -6.44
HRMS (ESI): calcd for [C₂₁H₂₅O₆P + Na]⁺ 427.1281, found 427.1281

Main product 12y (diastereo mixture): 2-Indanyl methyl-2,3,4-tri-O-Bz-α-D-glucopyranoside-6-yl phenyl phosphate
Purification method: preparative TLC (Hexane:EtOAc = 3:2)
Colorless oil; IR (neat): 2956, 1730, 1601, 1491, 1279, 1210, 1025, 950, 757, 688 cm⁻¹
¹H NMR (400 MHz, CDCl₃): δ = 7.97 (d, J = 6.8 Hz, 2H), 7.92 (d, J = 7.6 Hz, 2H), 7.85 (d, J = 7.6 Hz, 2H), 7.51 (t, J = 7.4 Hz, 2H), 7.44-7.34 (m, 5H), 7.32-7.25 (m, 4H), 7.23-7.13 (m, 7H), 6.14 (dd, J = 9.4, 9.4 Hz, 1H), 6.12 (dd, J = 9.4, 9.4 Hz, 1H), 5.52 (dd, J = 9.2, 9.2 Hz, 1H), 5.51 (dd, J = 9.2, 9.2 Hz, 1H), 5.38-5.34 (m, 1H), 5.26-5.14 (m, 2H), 4.34-4.24 (m, 3H), 3.41 (s, 3H), 3.38 (s, 3H), 3.31-3.10 (m, 4H)
¹³C NMR (125 MHz, CDCl₃): δ = 165.8, 165.7, 165.2, 139.6, 133.5, 133.4, 133.1, 129.92, 129.87, 129.7, 128.45, 128.42, 128.3, 126.9, 125.1 (d, J = 4.8 Hz), 124.6, 120.11 (d, J = 4.7 Hz), 120.06 (d, J = 4.8 Hz), 96.9 (d, J = 1.9 Hz), 80.44 (d, J = 5.8 Hz), 80.38 (d, J = 6.6 Hz), 71.9 (d, J = 4.8 Hz), 70.3, 69.1, 69.0, 68.4 (d, J = 3.8 Hz), 68.3 (d, J = 3.8 Hz), 66.6 (d, J = 5.7 Hz), 66.5 (d, J = 5.8 Hz), 55.6 (d, J = 2.9 Hz), 40.7 (d, J = 4.7 Hz)
³¹P NMR (162 MHz, CDCl₃): δ = -6.46, -6.51
HRMS (ESI): calcd for [C₄₃H₃₉O₁₂P + Na]⁺ 801.2071, found 801.2071

Main product 13y (diastereo mixture): 1,2-O-Dipalmitoyl-glycerol-3-yl ethyl 2-indanyl phosphate
Purification method: preparative TLC (Hexane:EtOAc = 7:3)
Colorless oil; IR (neat): 2924, 2853, 1744, 1276, 1035, 742 cm⁻¹
¹H NMR (400 MHz, CDCl₃): δ = 7.24-7.22 (m, 2H), 7.20-7.17 (m, 2H), 5.25-5.22 (m, 2H), 4.32 (dd, J = 4.0, 12.0 Hz, 1H), 4.18-4.08 (m, 4H), 3.29 (dd, J = 6.0, 16.6 Hz, 2H), 3.16 (dd, J = 3.4, 16.6 Hz, 2H), 2.31 (t, J = 7.6 Hz, 2H), 2.30 (t, J = 7.6 Hz, 2H), 1.62-1.56 (m, 4H), 1.35-1.25 (m, 51H), 0.88 (t, J = 6.9 Hz, 6H)
¹³C NMR (125 MHz, CDCl₃): δ = 173.2, 172.8, 139.7, 126.9, 124.6, 79.4 (d, J = 5.7 Hz), 69.4 (d, J = 7.6 Hz), 65.1 (d, J = 5.8 Hz), 64.1 (d, J = 4.7 Hz), 61.7, 40.7 (d, J = 4.7 Hz), 34.1, 34.0, 31.9, 29.7, 29.6, 29.4, 29.35, 29.27, 29.11, 29.07, 24.8, 22.7, 16.1 (d, J = 6.7 Hz), 14.1
³¹P NMR (162 MHz, CDCl₃): δ = -1.13
HRMS (ESI): calcd for [C₄₆H₈₁O₈P + Na]⁺ 815.5561, found 815.5561

Main product 14y: O-(sec-Butyl) O-(2,3-dihydro-1H-inden-2-yl) O-phenyl phosphorothioate
Purification method: preparative TLC (Hexane:EtOAc = 4:1)
Colorless oil; IR (neat): 2873, 1593, 1489, 1207, 995, 931, 784, 742, 689 cm⁻¹
¹H NMR (400 MHz, CDCl₃): δ = 7.31 (dd, J = 8.08.0 Hz, 2H), 7.23-7.15 (m, 7H), 5.42 (ttd, J = 3.2, 6.4, 9.5 Hz, 1H), 4.67 (tqd, J = 2.4, 6.1, 6.2 Hz, 1H), 4.65 (tqd, J = 2.4, 6.1, 6.2 Hz, 1H), 3.34-3.22, (m, 2H), 3.22-3.09 (m, 2H), 1.68 (dq, J = 6.8, 7.2 Hz, 2H), 1.34 (d, J = 6.2 Hz, 3H), 1.29 (d, J = 6.2 Hz, 3H), 0.94 (t, J = 7.4 Hz, 3H), 0.90 (t, J = 7.4 Hz, 3H)
¹³C NMR (125 MHz, CDCl₃): δ = 150.8 (d, J = 7.6 Hz), 139.87, 139.85, 129.4, 126.8, 125.1, 124.62, 124.58, 121.14 (d, J = 4.7 Hz), 121.11 (d, J = 4.8 Hz), 80.38 (d, J = 5.7 Hz), 80.37 (d, J = 5.7 Hz), 79.22 (d, J = 6.7 Hz), 79.15 (d, J = 6.6 Hz), 40.5 (d, J = 5.7 Hz), 40.43 (d, J = 5.7 Hz), 40.41 (d, J = 5.7 Hz), 30.12 (d, J = 6.7 Hz), 30.05 (d, J = 7.6 Hz), 20.8 (d, J = 3.8 Hz), 20.7 (d, J = 3.8 Hz), 9.5, 9.4
³¹P NMR (162 MHz, CDCl₃): δ = 62.3, 62.1.
HRMS (ESI): calcd for [C₁₉H₂₃O₃PS + Na]⁺ 385.0998, found 385.0998

Although preferred Examples of the present disclosure have been described above, the present disclosure is not limited to these Examples. Additions, omissions, substitutions, and other modifications of the configuration can be made without departing from the spirit of the present disclosure. The present disclosure is not limited by the foregoing description and is limited only by the scope of the appended claims.

### [Reference Signs List]

1, 100: Low reactor
11, 12, 13, 14, 15, 110, 120, 130, 140: Tank
21, 22, 23, 24, 210, 220, 230: Pump
31, 32, 33, 310, 320: Mixer
f1, f2, f3, f4, f5, f6, f7, f10, f20, f30, f40, f50: Flow channel

## Claims

1. A method for producing an asymmetric phosphoric acid triester in which the asymmetric phosphoric acid triester is synthesized using a flow reactor, the method comprising:
step (A1) of reacting phosphorus trichloride or phosphorus tribromide with a first hydroxy compound represented by general formula (H-1) below to synthesize a compound P1 represented by general formula (P-1) below;
step (B1) of reacting the compound P1 with a second hydroxy compound represented by general formula (H-2) below to synthesize a compound P2 represented by general formula (P-2) below;
step (C1) of reacting the compound P2 with a third hydroxy compound represented by general formula (H-3) below to synthesize a compound P3 represented by general formula (P-3) below; and
step (D1) of oxidizing the compound P3 to synthesize an asymmetric phosphoric acid triester,
wherein:
a reaction temperature in the steps (A1) and (B1) is -80°C or higher, and
in the steps (A1) and (B1), a flow rate of each of the phosphorus trichloride or phosphorus tribromide, the first hydroxy compound, and the second hydroxy compound flowing in a flow channel is 0.01 mL/min or more, 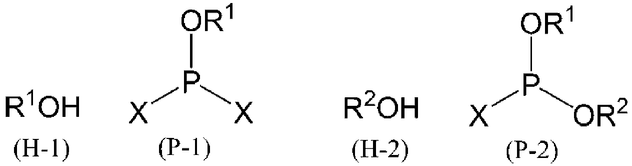 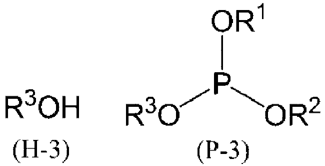 [R¹ to R³ are each independently an organic group, provided that R¹ to R³ are all different organic groups, and X is a bromine atom or a chlorine atom].

2. The method for producing an asymmetric phosphoric acid triester according to Claim 1,
wherein the reaction temperature in the steps (A1) and (Bl) is 0°C or higher.

3. The method for producing an asymmetric phosphoric acid triester according to Claim 1 or 2,
wherein, in the steps (A1) and (B1), the flow rate of each of the phosphorus trichloride or phosphorus tribromide, the first hydroxy compound, and the second hydroxy compound flowing in the flow channel is 1 mL/min or more.

4. The method for producing an asymmetric phosphoric acid triester according to any one of Claims 1 to 3, further comprising between the step (A1) and the step (B1), step (N1) of reacting the compound P1 with a base.

5. The method for producing an asymmetric phosphoric acid triester according to Claim 4,
wherein the base is one or more bases selected from the group consisting of pyridine, a pyridine derivative, imidazole, an imidazole derivative, and 1,4-diazabicyclo[2,2,2]octane.

6. The method for producing an asymmetric phosphoric acid triester according to Claim 4,
wherein the base is a compound represented by general formula (I-1) below: 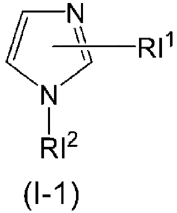 [in the formula, RI¹ and RI² are hydrogen atoms or hydrocarbon groups].

7. The method for producing an asymmetric phosphoric acid triester according to any one of Claims 1 to 6,
wherein, in the step (A1), an equivalent ratio of the phosphorus trichloride or phosphorus tribromide to R¹OH (phosphorus trichloride or phosphorus tribromide:R¹OH) in a reaction system is 0.1:1 to 10:1.

8. The method for producing an asymmetric phosphoric acid triester according to any one of Claims 1 to 7,
wherein the reaction temperature in the steps (A1) and (B1) is 20°C or higher and 40°C or lower.

9. The method for producing an asymmetric phosphoric acid triester according to any one of Claims 1 to 8,
wherein, in the steps (A1) and (B1), the flow rate of each of the phosphorus trichloride or phosphorus tribromide, the first hydroxy compound, and the second hydroxy compound flowing in the flow channel is 1 mL/min or more and 5 mL/min or less.

10. The method for producing an asymmetric phosphoric acid triester according to any one of Claims 1 to 9,
wherein, in the step (A 1), the flow rate of phosphorus trichloride or phosphorus tribromide and the flow rate of R'OH are different from each other.

11. The method for producing an asymmetric phosphoric acid triester according to Claim 10,
wherein, in the step (A1), the flow rate of phosphorus trichloride or phosphorus tribromide is 0.3 to 0.9 times the flow rate of R'OH.

12. The method for producing an asymmetric phosphoric acid triester according to Claim 10,
wherein, in the step (A1), the flow rate of R'OH is 0.3 to 0.9 times the flow rate of phosphorus trichloride or phosphorus tribromide.

13. A method for producing a symmetric phosphoric acid triester in which the symmetric phosphoric acid triester is synthesized using a flow reactor, the method comprising:
step (A2) of reacting phosphorus trichloride or phosphorus tribromide with a first hydroxy compound represented by general formula (H-1) below to synthesize compound P1 represented by general formula (P-1) below;
step (B2) of reacting the compound P1 with a second hydroxy compound represented by general formula (H-2) below to synthesize compound P4 represented by general formula (P-4) below; and
step (D2) of oxidizing the compound P4 to synthesize a symmetric phosphoric acid triester,
wherein a reaction temperature in the steps (A2) and (B2) is -80°C or higher, and
in the steps (A2) and (B2), a flow rate of each of the phosphorus trichloride or phosphorus tribromide, the first hydroxy compound, and the second hydroxy compound flowing in a flow channel is 0.01 mL/min or more, 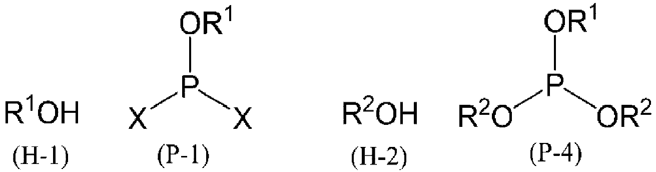 [in the formulae, R¹ and R² are each different organic groups, and X is a bromine atom or a chlorine atom].

14. The method for producing a symmetric phosphoric acid triester according to Claim 13, wherein the reaction temperature in the steps (A2) and (B2) is 0°C or higher.

15. The method for producing a symmetric phosphoric acid triester according to Claim 13 or 14,
wherein, in the steps (A2) and (B2), the flow rate of each of the phosphorus trichloride or phosphorus tribromide, the first hydroxy compound, and the second hydroxy compound flowing in the flow channel is 1 mL/min or more.

16. A method for producing a phosphoric acid ester in which the phosphoric acid ester is synthesized using a flow reactor, the method comprising:
step (A11) of reacting phosphorus trichloride or phosphorus tribromide with a first hydroxy compound represented by general formula (H1-1) below to synthesize compound P11 represented by general formula (P1-1) below;
step (B11) of reacting the compound P11 with a second hydroxy compound represented by general formula (H1-2) below to synthesize compound P12 represented by general formula (P1-2) below;
step (C11) of reacting the compound P12 with a third hydroxy compound represented by general formula (H1-3) below to synthesize compound P13 represented by general formula (P1-3) below; and
step (D11) of oxidizing the compound P13 to synthesize a phosphoric acid ester,
wherein a reaction temperature in the steps (A11) and (B11) is -80°C or higher, and
in the steps (A1 1) and (B11), a flow rate of each of the phosphorus trichloride or phosphorus tribromide, the first hydroxy compound, and the second hydroxy compound flowing in a flow channel is 0.01 mL/min or more, 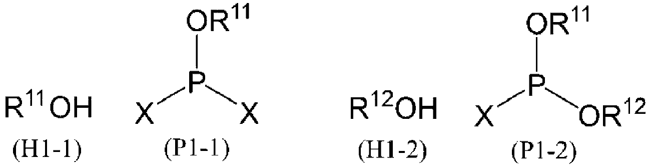 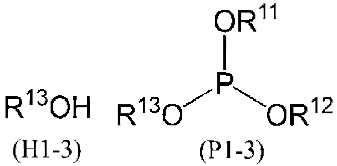 [in the formulae, R¹¹ to R¹³ are each independently a hydrogen atom or an organic group, provided that at least one of R¹¹ to R¹³ is an organic group and at least one of R¹¹ to R¹³ is a hydrogen atom, and X is a bromine atom or a chlorine atom].

17. A method for producing an organophosphorus compound in which the organophosphorus compound is synthesized using a flow reactor, the method comprising:
step (A01) of reacting phosphorus trichloride or phosphorus tribromide with a first hydroxy compound represented by general formula (H0-1) below to synthesize compound P01 represented by general formula (P0-1) below;
step (B01) of reacting the compound P01 with a second hydroxy compound represented by general formula (H0-2) below to synthesize compound P02 represented by general formula (P0-2) below; and
step (C01) of reacting the compound P02 with a third hydroxy compound represented by general formula (H0-3) below to synthesize compound P03 represented by general formula (P0-3) below,
wherein a reaction temperature in the steps (A01) and (B01) is -80°C or higher, and
in the steps (A01) and (B01), a flow rate of each of the phosphorus trichloride or phosphorus tribromide, the first hydroxy compound, and the second hydroxy compound in a flow channel is 0.01 mL/min or more, 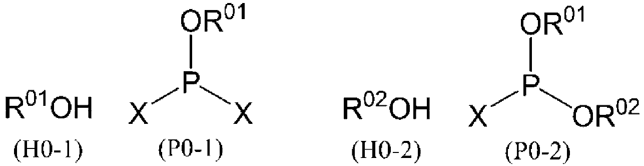 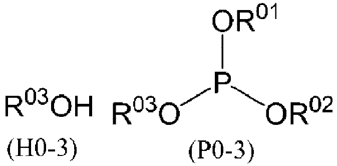 [in the formulae, R⁰¹ to R⁰³ are each independently a hydrogen atom or an organic group, provided that at least one of R⁰¹ to R⁰³ is an organic group, and when all of R⁰¹ to R⁰³ are organic groups, not all three of the organic groups are the same, and X is a bromine atom or a chlorine atom].

18. A method for producing an organophosphorus compound in which the organophosphorus compound is synthesized using a flow reactor, the method comprising:
step (AZ1) of reacting phosphorus trichloride or phosphorus tribromide with a first compound which is any of a hydroxy compound, an amine compound, or a thiol compound to synthesize compound PZ1 represented by general formula (PZ-1) below;
step (BZ1) of reacting the compound PZ1 with a second compound which is any of a hydroxy compound, an amine compound, or a thiol compound to synthesize compound PZ2 represented by general formula (PZ-2) below; and
step (CZ1) of reacting the compound PX2 with a third compound which is any of a hydroxy compound, an amine compound, or a thiol compound to synthesize compound PZ3 represented by general formula (PZ-3) below,
wherein a reaction temperature in the steps (AZ1) and (BZ1) is -80°C or higher, and
in the steps (AZ1) and (BZ1), a flow rate of each of the phosphorus trichloride or phosphorus tribromide, the first compound, and the second compound flowing in a flow channel is 0.01 mL/min or more, 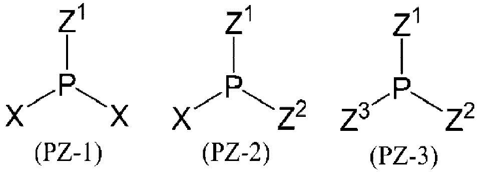 [in the formulae, Z¹ to Z³ are each independently a hydrogen atom, a group obtained by removing one hydrogen atom from a hydroxy compound, a group obtained by removing one hydrogen atom from an amine compound, or a group obtained by removing one hydrogen atom from a thiol compound, provided that not all three of Z¹ to Z³ are the same, and X is a bromine atom or a chlorine atom].
